# EUROPEAN PATENT SPECIFICATION

(11) **EP 3 851 888 B1**
(45) Date of publication and mention of the grant of the patent: **29.10.2025**
(21) Application number: 19860710.3
(22) Date of filing: 29.08.2019
(51) Int. Cl.: G02B 6/26, A61B 18/20, G02B 6/02

(54) **OPTICAL PROBE**
OPTISCHE SONDE
SONDE OPTIQUE

(30) Priority: 10.09.2018 JP 2018168432
(43) Date of publication of application: 21.07.2021
(73) Proprietor: FURUKAWA ELECTRIC CO., LTD., Chiyoda-ku Tokyo 100-8322 (JP)
(72) Inventor: WATANABE, Kengo, Tokyo 100-8322 (JP); MATSUSHITA, Shunichi, Tokyo 100-8322 (JP); NOMURA, Yoshiki, Tokyo 100-8322 (JP); TAKASAKA, Shigehiro, Tokyo 100-8322 (JP); IWAMA, Masaki, Tokyo 100-8322 (JP)
(74) Representative: SSM Sandmair
(86) International application number: PCT/JP2019/033979
(87) International publication number: WO 2020/054453

(56) References cited:
- WO-A1-01/11331
- WO-A1-2017/027695
- WO-A1-2017/115413
- WO-A1-89/11834
- CN-A- 104 382 548
- JP-A- 2007 171 676
- JP-A- 2008 036 153
- JP-A- 2010 268 961
- JP-A- 2014 094 122
- JP-A- 2016 512 616
- JP-A- S62 295 004
- JP-U- S6 430 474
- US-A- 5 242 438
- US-A- 5 537 499
- US-A- 5 836 941
- US-A1- 2017 311 806
- US-A1- 2018 049 806
- US-A1- 2018 168 729

## Description

### Field

The present invention relates to an optical probe.

### Background

A technology for performing treatment inside a body of a patient has been known. This kind of technology is used in, for example, a laser cautery device. The laser cautery device is a device that, for example, inserts a catheter in which an optical fiber is inserted into the body of the patient, outputs a laser beam for cautery from a distal end of the optical fiber to irradiate a target portion, such as an affected area, and performs treatment (see Patent Literature 1). A distal end side of the optical fiber inserted in the catheter may be referred to as an optical probe. In general, in the optical probe, a holder member for holding the optical fiber is mounted on the distal end side of the optical fiber.

### Citation List

### Patent Literature

Patent Literature 1: Japanese Unexamined Patent Application Publication No. 2017-535810 Reference is furthermore made to document WO01/11331 A1.

### Summary

### Technical Problem

For example, there may be a case in which it is desired to insert a catheter into a blood vessel of a patient and irradiate a site on a wall surface of the blood vessel with a beam, such as a laser beam. However, in this case, the optical fiber of the optical probe is located approximately parallel to the blood vessel; therefore, in some cases, even if a beam is output from the distal end of the optical fiber parallel to an optical axis of the optical fiber, the beam travels forward in the blood vessel and it becomes difficult to irradiate a target site, such as an affected area, with the beam. Therefore, it is preferable to change a traveling direction of the beam output from the optical fiber to a sideward direction and causes the beam to be oriented toward the wall surface of the blood vessel.

However, there is a limitation in the size of the optical probe that is inserted in to a body, such as a blood vessel, and therefore, it is difficult to adopt a complicated configuration as a means for changing a traveling direction of a beam. Further, if a means having a complicated configuration is adopted, in some cases, it may be difficult to manufacture the means with a small size. Furthermore, in the technology described in Patent Literature 1, a reflecting member is likely to rotate in a hollow hole, and it is difficult to fix a rotation direction, which is a problem.

The present invention has been conceived in view of the foregoing situation, and an object of the present invention is to provide an optical probe capable of changing a traveling direction of an output beam to a sideward direction.

### Solution to Problem

The invention and its preferred embodiments are defined in the appended set of claims. To resolve the above problem and attain the object, an optical probe according to an embodiment of the present invention includes: a holder member that is mounted on a distal end side of an optical fiber and holds the optical fiber; and a traveling direction changing unit that changes a traveling direction of an output beam to a sideward direction with respect to the optical fiber. Further, the traveling direction changing unit is a reflector that is joined to a part of a surface of the holder member and reflects the output beam.

An optical probe according to an embodiment of the present invention includes: a holder member that is mounted on a distal end side of an optical fiber and holds the optical fiber; and a traveling direction changing unit that changes a traveling direction of an output beam to a sideward direction with respect to the optical fiber. Further, the traveling direction changing unit is a part of the holder member and is configured with a reflecting portion that reflects the output beam.

An optical probe according to an embodiment of the present invention includes: a traveling direction changing unit that changes a traveling direction of a beam output from an optical fiber to a sideward direction with respect to the optical fiber. Further, the traveling direction changing unit is arranged on an end face of the optical fiber.

The optical probe according to an embodiment of the present invention further includes: a holder member that is mounted on a distal end side of the optical fiber and holds the optical fiber Further, the traveling direction changing unit is a diffraction grating that is arranged on the holder member and diffracts the output beam.

In the optical probe according to an embodiment of the present invention, the holder member includes an insertion hole and a diameter extending hole that communicates with the insertion hole and that has a larger inner diameter than the insertion hole, the optical fiber is inserted in the insertion hole, and a distal end surface of the optical fiber is located at a boundary of the insertion hole and the diameter extending hole or at a side of the diameter extending hole relative to the boundary.

In the optical probe according to an embodiment of the present invention, a distal end surface of the optical fiber from which the beam is output is inclined with respect to an optical axis of the optical fiber.

The optical probe according to an embodiment of the present invention further includes: a reflector that is arranged on a distal end surface of the optical fiber from which the beam is output, that transmits the beam, and that reflects a certain beam with a certain wavelength different from a wavelength of the beam.

The optical probe according to an embodiment of the present invention further includes: a Bragg grating that is arranged in a core portion of the optical fiber, that transmits the beam, and that reflects a certain beam with a certain wavelength different from a wavelength of the beam.

In the optical probe according to an embodiment of the present invention, a distal end surface of the optical fiber from which the beam is output is inclined with respect to an optical axis of the optical fiber, and the traveling direction changing unit is a reflector that is arranged on the distal end surface and that reflects the beam.

The optical probe according to an embodiment of the present invention further includes: a holder member that is mounted on a distal end side of the optical fiber and that holds the optical fiber. Further, the holder member includes an insertion hole and an opening hole that communicates with the insertion hole and that is opened on a side surface with respect to a direction in which the insertion hole is extended, the optical fiber is inserted in the insertion hole of the holder member, the distal end surface protrudes to an inside of the opening hole, and the distal end surface of the optical fiber is oriented to a side opposite to an opening side of the opening hole.

In the optical probe according to an embodiment of the present invention, the holder member has an approximately cylindrical outer shape.

In the optical probe according to an embodiment of the present invention, the reflector or the Bragg grating that reflects the certain beam with the certain wavelength different from the wavelength of the beam has reflectivity of 4% or higher with respect to the certain beam with the certain wavelength different from the wavelength of the beam.

In the optical probe according to an embodiment of the present invention, the reflector or the Bragg grating that reflects the certain beam with the certain wavelength different from the wavelength of the beam has reflectivity of 40% or higher with respect to the certain beam with the certain wavelength different from the wavelength of the beam.

In the optical probe according to an embodiment the present invention, the certain wavelength of the certain beam different from the beam is separated by 3 nanometers or more from the wavelength of the beam.

The optical probe according to an embodiment of the present invention further includes: a plurality of reflectors or Bragg gratings that reflect a plurality of beams with wavelengths different from the wavelength of the beam.

In the optical probe according to an embodiment the present invention, the wavelength of the beam belongs to a 980-nanometer wavelength range, and the certain beam with the certain wavelength different from the beam belongs to one of a visible region, an O band, and a C band.

In the optical probe according to an embodiment the present invention, a core diameter of the optical fiber Advantageous Effects of Invention

According to the present invention, it is possible to realize an optical probe capable of changing a traveling direction of an output beam to a sideward direction.

### Brief Description of Drawings

FIG. 1 is a schematic diagram illustrating an overall configuration of an optical probe according to a first embodiment.
FIG. 2 is a schematic diagram illustrating an overall configuration of an optical probe according to a second embodiment.
FIG. 3 is a schematic diagram illustrating an overall configuration of an optical probe according to a third embodiment.
FIG. 4 is a schematic diagram illustrating an overall configuration of an optical probe according to a fourth embodiment.
FIG. 5 is a diagram for explaining one example of a method of manufacturing the optical probe illustrated in FIG. 2.
FIG. 6 is a diagram for explaining one example of a method of manufacturing the optical probe illustrated in FIG. 3.
FIG. 7 is a diagram for explaining another example of the method of manufacturing the optical probe illustrated in FIG. 2.
FIG. 8 is a schematic diagram illustrating an overall configuration of an optical probe according to a fifth embodiment.
FIG. 9 is a schematic diagram illustrating an overall configuration of an optical probe according to a sixth embodiment.
FIG. 10 is a diagram for explaining one example of a method of manufacturing the optical probe according to the fifth embodiment.
FIG. 11A is a diagram for explaining an example of a shape of a reflecting surface.
FIG. 11B is a diagram for explaining an example of the shape of the reflecting surface.
FIG. 11C is a diagram for explaining an example of the shape of the reflecting surface.
FIG. 12A is a schematic diagram illustrating an overall configuration of an optical probe according to a seventh embodiment.
FIG. 12B is a schematic diagram illustrating an overall configuration of the optical probe according to the seventh embodiment.
FIG. 13 is a schematic diagram illustrating an overall configuration of an optical probe according to an eighth embodiment.
FIG. 14 is a schematic diagram illustrating an overall configuration of an optical probe according to a ninth embodiment.
FIG. 15 is a diagram for explaining one example of a method of manufacturing the optical probe according to the seventh embodiment.
FIG. 16A is a schematic diagram illustrating an overall configuration of an optical probe according to a tenth embodiment.
FIG. 16B is a schematic diagram illustrating an overall configuration of the optical probe according to the tenth embodiment.
FIG. 17 is a schematic diagram illustrating an overall configuration of an optical probe according to an eleventh embodiment.
FIG. 18 is a schematic diagram illustrating an overall configuration of an optical probe according to a twelfth embodiment.
FIG. 19A is a schematic diagram illustrating an overall configuration of an optical probe according to a thirteenth embodiment.
FIG. 19B is a schematic diagram illustrating an overall configuration of the optical probe according to the thirteenth embodiment.
FIG. 20 is a diagram for explaining one example of a method of manufacturing the optical probe illustrated in FIGS. 19A and 19B.
FIG. 21 is a schematic diagram illustrating an overall configuration of a first configuration example of an optical fiber.
FIG. 22 is a schematic diagram illustrating an overall configuration of a second configuration example of an optical fiber.
FIG. 23 is a schematic diagram illustrating an overall configuration of an optical probe according to a fourteenth embodiment.
FIG. 24 is a schematic diagram illustrating an overall configuration of a third configuration example of an optical fiber.

### Description of Embodiments

Embodiments of the present invention will be described in detail below with reference to the accompanying drawings. The present invention is not limited by the embodiments described below. Further, in the description of the drawings, the same or corresponding components are denoted by the same reference symbols appropriately, and explanation thereof will be omitted appropriately. Furthermore, the drawings are schematic, and dimensional relations among the components, ratios among the components, and the like may be different from the actual ones. Moreover, the drawings may include portions that have different dimensional relations or ratios.

### First Embodiment

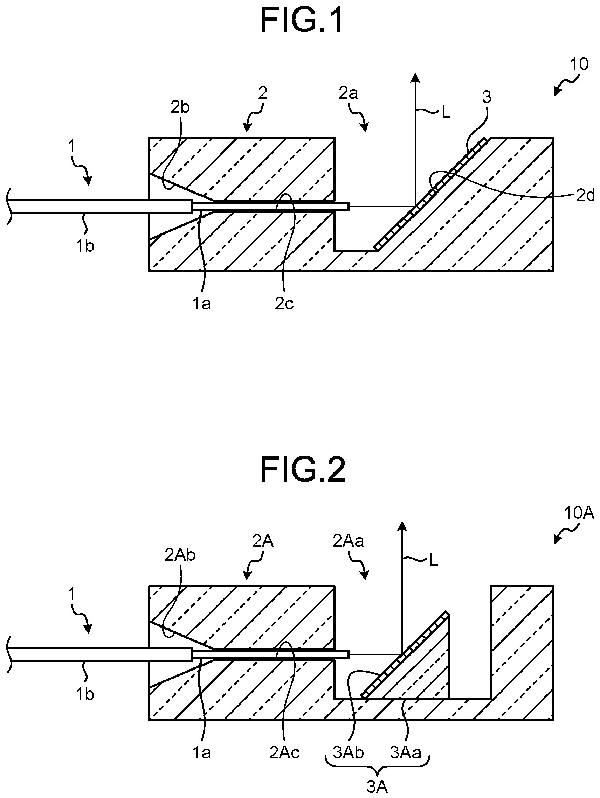

FIG. 1 is a schematic diagram illustrating an overall configuration of an optical probe according to a first embodiment. An optical probe 10 is used in, for example, a laser cautery device for treatment and is inserted into a lumen of a catheter.

The optical probe 10 includes an optical fiber 1, a holder member 2, and a reflecting coating 3. The optical fiber 1 includes a glass optical fiber 1a having a core portion and a cladding portion, and a covering 1b that is formed on an outer circumference of the glass optical fiber 1a. In the optical fiber 1, the covering 1b is removed on a distal end side, and a predetermined length of the glass optical fiber 1a is exposed. The optical fiber 1 transmits laser beam L in the glass optical fiber 1a and outputs the laser beam L from a distal end thereof. The laser beam L is, for example, a laser beam for cautery, and a wavelength thereof belongs to, for example, a 980-nanometer (nm) wavelength range. The 980-nm wavelength range is, for example, a wavelength range of 900 nm to 1000 nm. A proximal end side of the optical fiber 1 is optically connected to a laser beam source that generates the laser beam L.

The glass optical fiber 1a is, for example, a multi-mode optical fiber, and has a step-index (SI) or graded-index (GI) refractive index profile. The glass optical fiber 1a with a core diameter of 65 micrometers (µm) or larger is appropriate for transmission of high-power beam, but the glass optical fiber 1a is not specifically limited.

The holder member 2 is a member for holding the optical fiber 1, and is mounted on the distal end side of the optical fiber 1. The holder member 2 has an approximately cylindrical outer shape and is made of glass in the present embodiment, but a constituent material is not limited to glass, but may be resin, ceramic, plastic or the like. A diameter of the holder member 2 is, for example, approximately 1 to 2 millimeters (mm) or smaller. Meanwhile, the holder member 2 has an approximately cylindrical outer shape, but may have an approximately polygonal prism outer shape.

The holder member 2 includes an opening hole 2a, an optical fiber input hole 2b, and an insertion hole 2c. The optical fiber input hole 2b is formed so as to extend from an end face of the holder member 2 on the left side in the figure along a cylindrical central shaft of the holder member 2 or the vicinity of the cylindrical central shaft, and has a gradually reduced inner diameter. The insertion hole 2c communicates with the optical fiber input hole 2b on a distal end side (on the right side in the figure) of the optical fiber input hole 2b, and is formed so as to extend along the cylindrical central shaft of the holder member 2 or the vicinity of the cylindrical central shaft. An inner diameter of the insertion hole 2c is slightly larger than an outer diameter of the glass optical fiber 1a. The opening hole 2a communicates with the insertion hole 2c, and is opened on a side surface in a direction in which the insertion hole 2c extends, that is, on a cylindrical outer periphery of the holder member 2.

The optical fiber 1 is inserted into the holder member 2 from the optical fiber input hole 2b, and is held by being fixed with an adhesive or the like. The exposed glass optical fiber 1a is inserted into the insertion hole 2c, and a distal end thereof protrudes to the inside of the opening hole 2a. The glass optical fiber 1a is bonded to an inner surface of the insertion hole 2c with an adhesive or the like. Further, a part of the optical fiber 1 input in the optical fiber input hole 2b, that is, a distal end portion or the like of the covering 1b, is bonded to an inner surface of the optical fiber input hole 2b with an adhesive or the like.

The holder member 2 includes an inclined surface 2d at a position facing a distal end surface of the optical fiber 1, that is, a distal end surface of the glass optical fiber 1a, inside the opening hole 2a. The reflecting coating 3 as a reflector is arranged on the inclined surface 2d. The reflecting coating 3 is configured with a metal film, a dielectric multi-layer or the like, and is arranged on the inclined surface 2d by well-known vapor deposition, a chemical vapor deposition (CVD) method or the like. Meanwhile, the reflecting coating 3 may be separately manufactured and arranged by being attached to the inclined surface 2d with an adhesive, an adhesive material or the like. The inclined surface 2d and a reflecting surface of the reflecting coating 3 are inclined by approximately 45 degrees with respect to an optical axis of the optical fiber 1.

The reflecting coating 3 functions as a traveling direction changing means that changes a traveling direction of the laser beam L output from the optical fiber 1 to a sideward direction with respect to the optical fiber 1. In the present embodiment, the reflecting coating 3 reflects the laser beam L that travels along the optical axis of the optical fiber 1 after being output, and changes the traveling direction of the laser beam L by approximately 90 degrees.

In the optical probe 10, the reflecting coating 3 arranged on the holder member 2 changes the traveling direction of the laser beam L output from the optical fiber 1 by approximately 90 degrees to change the traveling direction to a lateral side. According to the optical probe 10, it is possible to change the traveling direction of the laser beam L with a simple, small, and easily manufacturable configuration. In particular, the reflecting coating 3 is arranged inside the opening hole 2a without protruding to an outer diameter side of the holder member 2, so that it is possible to reduce an outer diameter of the optical probe 10.

### Second Embodiment

FIG. 2 is a schematic diagram illustrating an overall configuration of an optical probe according to a second embodiment. An optical probe 10A includes the optical fiber 1, a holder member 2A, and a reflecting member 3A.

The holder member 2A is a member for holding the optical fiber 1, and is mounted on the distal end side of the optical fiber 1. The holder member 2A has an approximately cylindrical outer shape and is made of glass in the present embodiment, but a constituent material is not limited to glass. A diameter of the holder member 2A is, for example, approximately 1 to 2 mm or smaller.

The holder member 2A includes an opening hole 2Aa, an optical fiber input hole 2Ab, and an insertion hole 2Ac. The optical fiber input hole 2Ab and the insertion hole 2Ac respectively have the same configurations as the optical fiber input hole 2b and the insertion hole 2c in FIG. 1, and therefore, explanation thereof will be omitted appropriately. The opening hole 2Aa communicates with the insertion hole 2Ac, and is opened on a side surface in a direction in which the insertion hole 2Ac extends, that is, on a cylindrical outer periphery of the holder member 2A.

The optical fiber 1 is held by the holder member 2A in the same manner as in the optical probe 10 in FIG. 1.

The reflecting member 3A is arranged at a position facing the distal end surface of the optical fiber 1 inside the opening hole 2Aa. The reflecting member 3A includes a member 3Aa that is made of glass or the like and that has a certain shape, such as a triangular prism or a tetrahedron, and a reflecting coating 3Ab that is arranged on one surface of the member 3Aa. The one surface of the member 3Aa and a reflecting surface of the reflecting coating 3Ab are inclined by approximately 45 degrees with respect to the optical axis of the optical fiber 1. The reflecting coating 3Ab is configured with a metal film, a dielectric multi-layer or the like, and is arranged on the member 3Aa by well-known vapor deposition, a CVD method or the like. Meanwhile, the reflecting coating 3Ab may be separately manufactured and arranged by being attached to the member 3Aa with an adhesive, an adhesive material or the like. Further, the member 3Aa is fixed to the inside of the opening hole 2a of the holder member 2A with an adhesive or the like.

The reflecting coating 3Ab functions as the traveling direction changing means similarly to the reflecting coating 3 in the optical probe 10 in FIG. 1. The reflecting coating 3Ab as a reflector is joined to a part of a surface of the holder member 2A. In the present embodiment, the reflecting coating 3Ab reflects the laser beam L that travels along the optical axis of the optical fiber 1 after being output, and changes the traveling direction of the laser beam L by approximately 90 degrees.

According to the optical probe 10A, it is possible to change the traveling direction of the laser beam L with a simple, small, and easily manufacturable configuration. In particular, the reflecting coating 3Ab is arranged inside the opening hole 2Aa without protruding to an outer diameter side of the holder member 2A, so that it is possible to reduce an outer diameter of the optical probe 10A.

### Third Embodiment

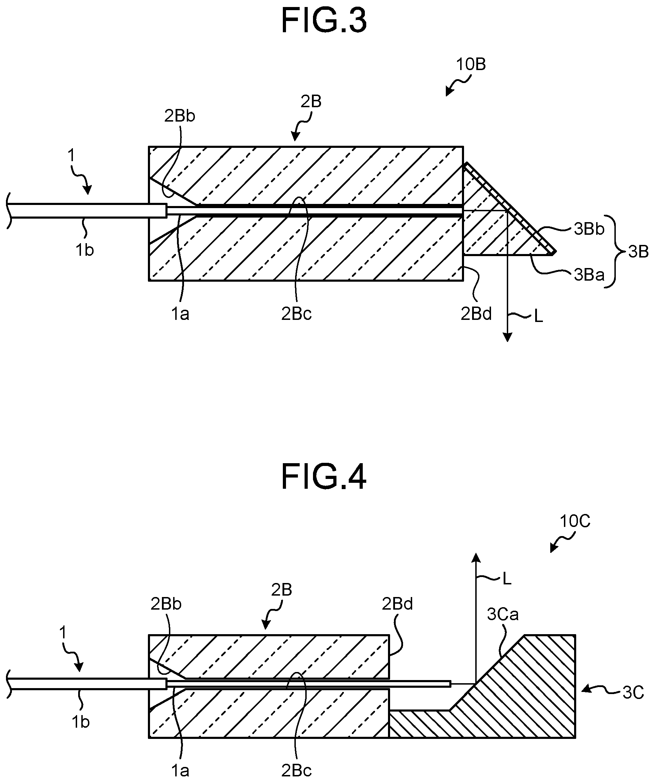

FIG. 3 is a schematic diagram illustrating an overall configuration of an optical probe according to a third embodiment. An optical probe 10B includes the optical fiber 1, a holder member 2B, and a reflecting member 3B.

The holder member 2B is mounted on the distal end side of the optical fiber 1. The holder member 2B has an approximately cylindrical outer shape and is made of glass in the present embodiment, but a constituent material is not limited to glass. A diameter of the holder member 2B is, for example, approximately 1 to 2 mm or smaller.

The holder member 2B includes an optical fiber input hole 2Bb and an insertion hole 2Bc. The optical fiber input hole 2Bb has the same configuration as the optical fiber input hole 2b in FIG. 1, and therefore, explanation thereof will be omitted appropriately. The insertion hole 2Bc communicates with the optical fiber input hole 2Bb on a distal end side of the optical fiber input hole 2Bb, and is formed so as to extend along a cylindrical central shaft of the holder member 2B or the vicinity of the cylindrical central shaft. An inner diameter of the insertion hole 2Bc is slightly larger than an outer diameter of the glass optical fiber 1a. The insertion hole 2Bc penetrates to an end face 2Bd of the holder member 2B that is located on the right side in the figure.

The optical fiber 1 is held by the holder member 2B in the same manner as in the optical probe 10 in FIG. 1. Meanwhile, the distal end surface of the optical fiber 1 is located on the same plane of the end face 2Bd of the holder member 2B or on a side that is slightly closer to the optical fiber input hole 2Bb than the end face 2Bd.

The reflecting member 3B is arranged on the end face 2Bd of the holder member 2B. The reflecting member 3B includes a member 3Ba that has a certain shape, such as a triangular prism or a tetrahedron, and a reflecting coating 3Bb that is arranged on one surface of the member 3Ba. The member 3Ba is made of a material, such as glass, that transmits the laser beam L. The one surface of the member 3Ba and a reflecting surface of the reflecting coating 3Bb are inclined by approximately 45 degrees with respect to the optical axis of the optical fiber 1. The reflecting coating 3Bb is configured with a metal film, a dielectric multi-layer or the like, and is arranged on the member 3Ba by well-known vapor deposition, a CVD method or the like. Meanwhile, the reflecting coating 3Bb may be separately manufactured and arranged by being attached to the member 3Ba with an adhesive, an adhesive material or the like. Further, the member 3Ba is fixed to the end face 2Bd of the holder member 2B with an adhesive or the like. Furthermore, it is preferable to form an antireflection coating on a surface of the member through which the laser beam L passes, such as the end face 2Bd of the holder member 2B or a surface of the member 3Ba that comes in contact with the holder member 2B.

The reflecting coating 3Bb functions as the traveling direction changing means similarly to the reflecting coating 3 in the optical probe 10 in FIG. 1. The reflecting coating 3Bb as a reflector is joined to a part of a surface of the holder member 2B. In the present embodiment, the reflecting coating 3Bb reflects the laser beam L that travels along the optical axis of the optical fiber 1 after being output, and changes the traveling direction of the laser beam L by approximately 90 degrees.

According to the optical probe 10B, it is possible to change the traveling direction of the laser beam L with a simple, small, and easily manufacturable configuration. In particular, the reflecting coating 3Bb is arranged without protruding to an outer diameter side of the holder member 2B, so that it is possible to reduce an outed diameter of the optical probe 10B.

Furthermore, by forming a refractive index profile on the member 3Ba through which the laser beam L passes, it is possible to collect, diffuse, or collimate the laser beam L. With this configuration, it is possible to control a power profile of the laser beam L in an irradiation target portion, such as an affected area.

### Fourth Embodiment

FIG. 4 is a schematic diagram illustrating an overall configuration of an optical probe according to a fourth embodiment. An optical probe 10C includes the optical fiber 1, the holder member 2B, and a reflecting member 3C. The optical fiber 1 has the same configuration as the optical fiber in FIG. 1, and therefore, explanation thereof will be omitted appropriately.

The holder member 2B has the same configuration as the holder member 2B in FIG. 3, and therefore, explanation thereof will be omitted appropriately. The optical fiber 1 is held by the holder member 2B in the same manner as in the optical probe 10B in FIG. 3. However, in the optical probe 10C, the distal end surface of the optical fiber 1 protrudes from the end face 2Bd of the holder member 2B.

The reflecting member 3C is arranged on the end face 2Bd of the holder member 2B. The reflecting member 3C is configured with a material, such as metal, that reflects the laser beam L. The reflecting member 3C can be manufactured by, for example, machining by mechanical processing, molding using a die, powder burning or the like. The reflecting member 3C includes a reflecting surface 3Ca that is inclined by approximately 45 degrees with respect to the optical axis of the optical fiber 1. The reflecting member 3C is fixed to the end face 2Bd of the holder member 2B with an adhesive or the like. Meanwhile, a shape formed by the holder member 2B and the reflecting member 3C is approximately the same as the shape of the holder member 2 in FIG. 1.

The reflecting surface 3Ca functions as the traveling direction changing means similarly to the reflecting coating 3 in the optical probe 10 in FIG. 1. The reflecting member 3C as a reflector is joined to a part of a surface of the holder member 2B. In the present embodiment, the reflecting surface 3Ca reflects the laser beam L that travels along the optical axis of the optical fiber 1 after being output, and changes the traveling direction of the laser beam L by approximately 90 degrees.

According to the optical probe 10C, it is possible to change the traveling direction of the laser beam L with a simple, small, and easily manufacturable configuration. In particular, the reflecting member 3C is arranged without protruding to the outer diameter side of the holder member 2B, so that it is possible to reduce an outer diameter of the optical probe 10C.

Meanwhile, in the present embodiment, the reflecting member 3C is made of metal, but it may be possible to arrange, instead of the reflecting member 3C, a reflecting member that is made with a material, such as glass, resin, ceramic, or plastic, that does not reflect the laser beam L or that has low reflectivity, and that has approximately the same shape as that of the reflecting member 3C. In this case, it is preferable to arrange, on the reflecting member, an inclined surface that is inclined by approximately 45 degrees with respect to the optical axis of the optical fiber 1, and arrange a reflecting coating that is made of metal or a dielectric multi-layer on the inclined surface. Furthermore, it may be possible to fix the holder member 2B and the reflecting member by welding or optical contact that is a method of joining highly-precisely polished surfaces by intermolecular forces, depending on the material of the reflecting member.

### Manufacturing Method

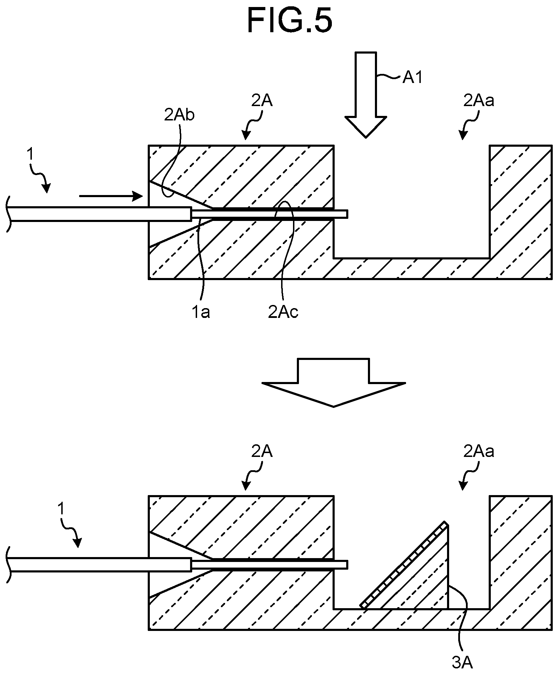

One example of a method of manufacturing the optical probe 10A according to the second embodiment illustrated in FIG. 2 will be described below with reference to FIG. 5. First, the optical fiber 1 is inserted into the holder member 2A from the optical fiber input hole 2Ab and is inserted in the insertion hole 2Ac, and a relative position of the optical fiber 1 with respect to the holder member 2A is adjusted while monitoring a position of a distal end of the optical fiber 1 (a distal end of the glass optical fiber 1a) in a direction of an arrow A1. Then, after the relative position reaches a predetermined position, the holder member 2A and the optical fiber 1 are fixed to each other. Subsequently, the reflecting member 3A is fixed to a predetermined position on the holder member 2A to which the optical fiber 1 is fixed. Here, the predetermined position is a predetermined position inside the opening hole 2Aa of the holder member 2A. The predetermined position may be finely adjusted such that an optical path of the reflected laser beam L matches a desired optical path with regard to the relative position with respect to the optical fiber 1. Furthermore, it may be possible to first fix the member 3Aa of the reflecting member 3A to the holder member 2A, and thereafter arrange the reflecting coating 3Ab on the member 3Aa.

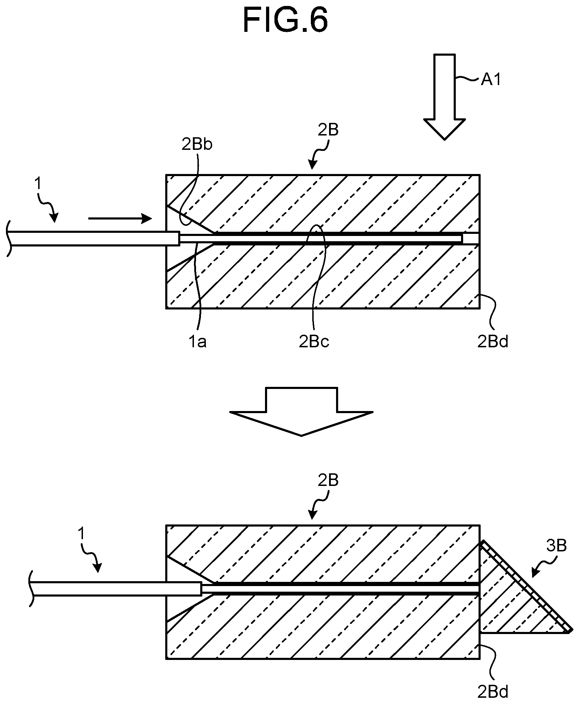

Next, one example of a method of manufacturing the optical probe 10B according to the third embodiment illustrated in FIG. 3 will be described with reference to FIG. 6. First, the optical fiber 1 is inserted into the holder member 2B from the optical fiber input hole 2Bb and is inserted in the insertion hole 2Bc, and a relative position of the optical fiber 1 with respect to the holder member 2B is adjusted while monitoring the position of the distal end of the optical fiber 1 (the distal end of the glass optical fiber 1a) in the direction of the arrow A1. Then, after the relative position reaches a predetermined position, the holder member 2B and the optical fiber 1 are fixed to each other. Subsequently, the reflecting member 3B is fixed to a predetermined position on the holder member 2B to which the optical fiber 1 is fixed. Here, the predetermined position is a predetermined position on the end face 2Bd of the holder member 2B. The predetermined position may be finely adjusted such that the optical path of the reflected laser beam L matches a desired optical path with regard to the relative position with respect to the optical fiber 1. Furthermore, it may be possible to first fix the member 3Ba of the reflecting member 3B to the holder member 2B, and thereafter arrange the reflecting coating 3Bb on the member 3Ba.

The optical probes 10 and 10C according to the first and the fourth embodiments illustrated in FIGS. 1 and 4 can easily be manufactured in the same manner as the simple manufacturing methods as illustrated in FIGS. 5 and 6.

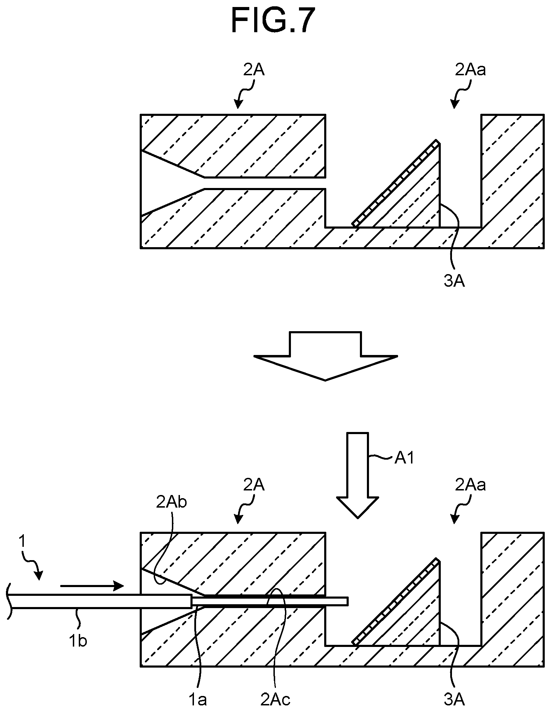

Next, another example of the method of manufacturing the optical probe 10A according to the second embodiment illustrated in FIG. 2 will be described with reference to FIG. 7. First, the reflecting member 3A is fixed at a predetermined position inside the opening hole 2Aa of the holder member 2A. Subsequently, the optical fiber 1 is inserted into the holder member 2A from the optical fiber input hole 2Ab and is inserted in the insertion hole 2Ac, and a relative position of the optical fiber 1 with respect to the holder member 2A is adjusted while monitoring the position of the distal end of the optical fiber 1 in the direction of the arrow A1. Then, after the relative position reaches a predetermined position, the holder member 2A and the optical fiber 1 are fixed to each other. Meanwhile, the position at which the optical fiber 1 is fixed may be finely adjusted such that the optical path of the reflected laser beam L matches a desired optical path with regard to the relative position with respect to the reflecting member 3A.

The optical probes 10, 10B, and 10C according to the first, the third, and the fourth embodiments illustrated in FIGS. 1, 3, and 4 can easily be manufactured in the same manner as the simple manufacturing method as illustrated in FIG. 7.

### Fifth Embodiment

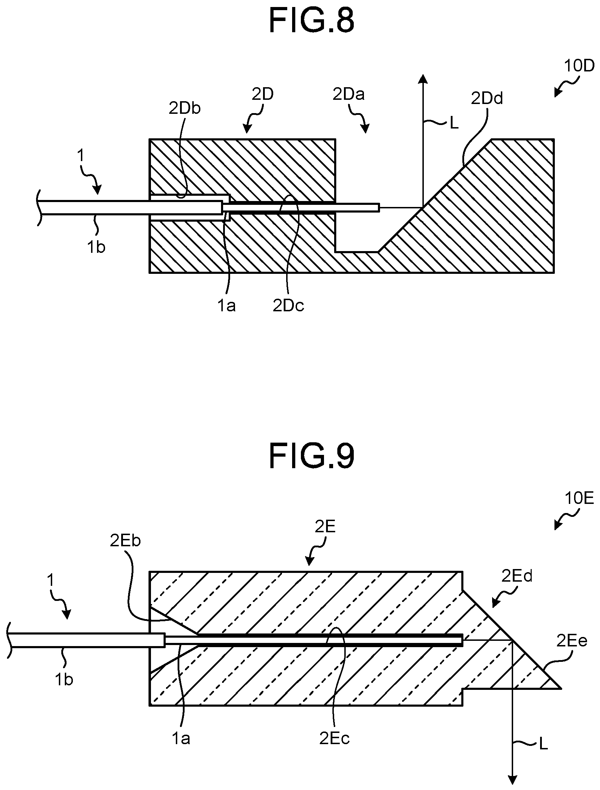

FIG. 8 is a schematic diagram illustrating an overall configuration of an optical probe according to a fifth embodiment. An optical probe 10D includes the optical fiber 1 and a holder member 2D. The optical fiber 1 has the same configuration as the optical fiber in FIG. 1, and therefore, explanation thereof will be omitted appropriately.

The holder member 2D is mounted on the distal end side of the optical fiber 1. The holder member 2D has an approximately cylindrical outer shape and is made of a material, such as metal, that reflects the laser beam L. A diameter of the holder member 2D is, for example, approximately 1 to 2 mm or smaller. The holder member 2D may be manufactured by, for example, machining by mechanical processing, molding using a die, powder burning or the like.

The holder member 2D includes an opening hole 2Da, an optical fiber input hole 2Db, and an insertion hole 2Dc. The optical fiber input hole 2Db is formed so as to extend from an end face of the holder member 2D along a cylindrical central shaft of the holder member 2D or the vicinity of the cylindrical central shaft, and has an approximately constant inner diameter; however, the inner diameter may be gradually reduced. The insertion hole 2Dc communicates with the optical fiber input hole 2Db on a distal end side of the optical fiber input hole 2Db (on the right side in the figure), and is formed so as to extend along the cylindrical central shaft of the holder member 2D or the vicinity of the cylindrical central shaft. An inner diameter of the insertion hole 2Dc is slightly larger than the outer diameter of the glass optical fiber 1a. The opening hole 2Da communicates with the insertion hole 2Dc, and is opened on a side surface in a direction in which the insertion hole 2Dc extends, that is, on a cylindrical outer periphery of the holder member 2D.

The optical fiber 1 is held by the holder member 2D in the same manner as in the optical probe 10 in FIG. 1.

In the holder member 2D, a reflecting surface 2Dd that forms an inner wall of the opening hole 2Da is arranged at a position facing the distal end surface of the optical fiber 1. The reflecting surface 2Dd is inclined by approximately 45 degrees with respect to the optical axis of the optical fiber 1.

The reflecting surface 2Dd is a part of the holder member 2D and is a reflecting portion that reflects the laser beam L1 output from the optical fiber 1. In the present embodiment, the traveling direction changing means is configured with the reflecting surface 2Dd. In other words, in the present embodiment, the reflecting surface 2Dd reflects the laser beam L that travels along the optical axis of the optical fiber 1 after being output, and changes the traveling direction of the laser beam L by approximately 90 degrees.

According to the optical probe 10D, it is possible to change the traveling direction of the laser beam L with a simple, small, and easily manufacturable configuration. In particular, the reflecting surface 2Dd is a part of the holder member 2D, so that it is possible to reduce an outer diameter of the optical probe 10D and reduce the number of use components.

### Sixth Embodiment

FIG. 9 is a schematic diagram illustrating an overall configuration of an optical probe according to a sixth embodiment. An optical probe 10E includes the optical fiber 1 and a holder member 2E. The optical fiber 1 has the same configuration as the optical fiber in FIG. 1, and therefore, explanation thereof will be omitted appropriately.

The holder member 2E is a member for holding the optical fiber 1, and is mounted on the distal end side of the optical fiber 1. The holder member 2E has an approximately cylindrical outer shape and is made of a material, such as glass, that transmits the laser beam L. A diameter of the holder member 2E is, for example, approximately 1 to 2 mm or smaller.

The holder member 2E includes an optical fiber input hole 2Eb, an insertion hole 2Ec, and a projection portion 2Ed. The optical fiber input hole 2Eb is formed so as to extend from an end face of the holder member 2E on the left side in the figure along a cylindrical central shaft of the holder member 2E or the vicinity of the cylindrical central shaft, and has a gradually reduced inner diameter. The insertion hole 2Ec communicates with the optical fiber input hole 2Eb on a distal end side of the optical fiber input hole 2Eb (on the right side in the figure), and is formed so as to extend along the cylindrical central shaft of the holder member 2E or the vicinity of the cylindrical central shaft. An inner diameter of the insertion hole 2Ec is slightly larger than the outer diameter of the glass optical fiber 1a. The projection portion 2Ed is formed, in the holder member 2E, on an end face opposite to the end face on which the optical fiber input hole 2Eb is formed. The projection portion 2Ed has a certain shape, such as a triangular prism or a tetrahedron.

The optical fiber 1 is held by the holder member 2E in the same manner as in the optical probe 10 in FIG. 1.

The projection portion 2Ed includes a reflecting surface 2Ee as one surface thereof. The reflecting surface 2Ee is inclined by approximately 45 degrees with respect to the optical axis of the optical fiber 1.

The reflecting surface 2Ee is a part of the holder member 2E and is a reflecting portion that reflects the laser beam L1 output from the optical fiber 1. In the present embodiment, the traveling direction changing means is configured with the reflecting surface 2Ee. In other words, in the present embodiment, the reflecting surface 2Ee reflects the laser beam L that travels along the optical axis of the optical fiber 1 after being output, and changes the traveling direction of the laser beam L by approximately 90 degrees.

According to the optical probe 10E, it is possible to change the traveling direction of the laser beam L with a simple, small, and easily manufacturable configuration. In particular, the reflecting surface 2Ee is a part of the holder member 2E, so that it is possible to reduce an outer diameter of the optical probe 10E and reduce the number of use components.

Furthermore, by forming a refractive index profile on a portion, such as the projection portion 2Ed, through which the laser beam L passes in the holder member 2E, it is possible to collect, diffuse, or collimate the laser beam L. With this configuration, it is possible to control a power profile of the laser beam L in an irradiation target portion, such as an affected area.

### Manufacturing Method

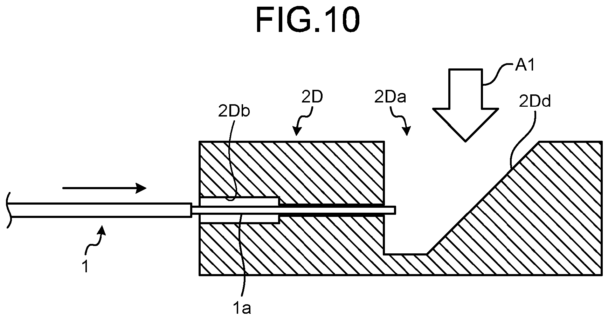

One example of a method of manufacturing the optical probe 10D according to the fifth embodiment illustrated in FIG. 8 will be described with reference to FIG. 10. First, the optical fiber 1 is inserted into the holder member 2D from the optical fiber input hole 2Db and is inserted in the insertion hole 2Dc, and the relative position of the optical fiber 1 with respect to the holder member 2D is adjusted while monitoring the position of the distal end of the optical fiber 1 (the distal end of the glass optical fiber 1a) in the direction of the arrow A1. Then, after the relative position reaches a predetermined position, the holder member 2D and the optical fiber 1 are fixed to each other. Meanwhile, the position at which the optical fiber 1 is fixed may be finely adjusted such that the optical path of the reflected laser beam L matches a desired optical path with regard to the relative position with respect to the reflecting surface 2Dd.

The optical probe 10E according to the sixth embodiment illustrated in FIG. 9 can easily be manufactured in the same manner as the simple manufacturing method as illustrated in FIG. 10.

### Shape of reflecting surface

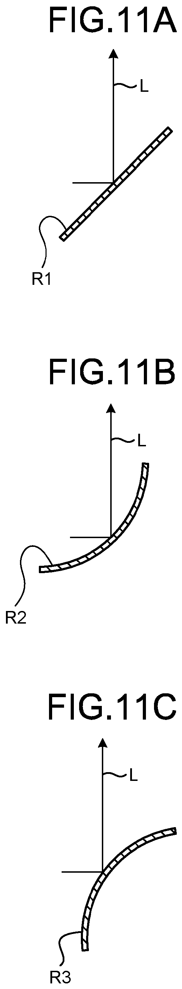

Here, the shape of the reflecting surface in each of the embodiments will be described. The reflecting surface for the laser beam L in each of the embodiments above and below is illustrated as a flat surface like a reflecting surface R1 in FIG. 11A, but may have a concave shape like a reflecting surface R2 in FIG. 11B or may have a convex shape like a reflecting surface R3 in FIG. 11C. In the case of the concave shape and the convex shape, a spherical shape, a paraboloidal shape, or other shapes may be adopted. By setting the shape of the reflecting surface as described above, it is possible to collect, diffuse, or collimate the laser beam L. With this configuration, it is possible to control a power profile of the laser beam L in an irradiation target portion, such as an affected area.

### Seventh Embodiment

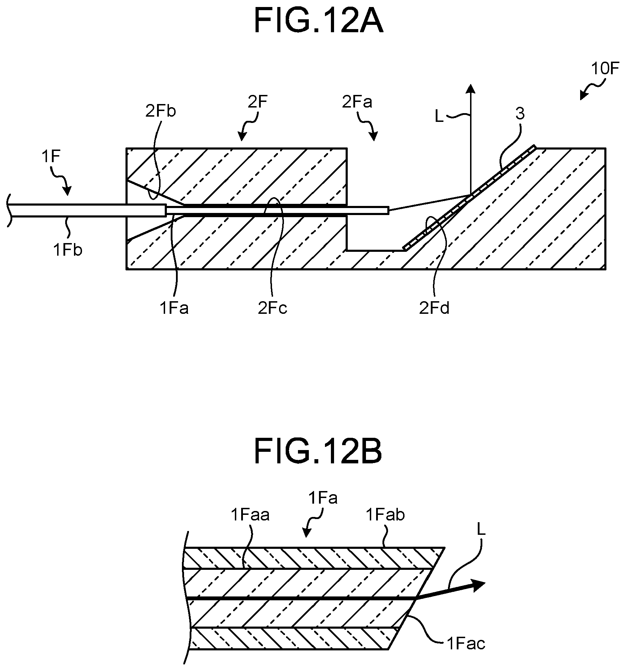

FIG. 12A and FIG. 12B are schematic diagrams illustrating an overall configuration of an optical probe according to a seventh embodiment. As illustrated in FIG. 12A, the optical probe 10F includes an optical fiber 1F, a holder member 2F, and the reflecting coating 3.

As illustrated in FIG. 12A and FIG. 12B, the optical fiber 1F includes a glass optical fiber 1Fa having a core portion 1Faa and a cladding portion 1Fab, and a covering 1Fb that is formed on an outer circumference of the glass optical fiber 1Fa. In the optical fiber 1F, the covering 1Fb is removed on a distal end side, and a predetermined length of the glass optical fiber 1Fa is exposed. The optical fiber 1F has the same configuration as the optical fiber 1 except that a distal end surface 1Fac from which the laser beam L is output is inclined with respect to an optical axis of the optical fiber 1F, that is, with respect to an optical axis of the glass optical fiber 1Fa, and therefore, explanation thereof will be omitted appropriately. In the optical fiber 1F, the distal end surface 1Fac is inclined, so that the laser beam L is output in an inclined direction with respect to the optical axis of the optical fiber 1F in accordance with an inclination angle. Meanwhile, the distal end surface 1Fac is inclined by approximately 10 degrees with respect to a plane perpendicular to the optical axis of the optical fiber 1F. The inclination angle as described above can easily be formed by a fiber cutter, mechanical polishing, chemical etching or the like.

The holder member 2F is mounted on a distal end side of the optical fiber 1F. The holder member 2F includes an opening hole 2Fa, an optical fiber input hole 2Fb, and an insertion hole 2Fc. The opening hole 2Fa, the optical fiber input hole 2Fb and the insertion hole 2Fc have the same configurations as the opening hole 2a, the optical fiber input hole 2b, and the insertion hole 2c, respectively, illustrated in FIG. 1, and therefore, explanation thereof will be omitted appropriately.

The optical fiber 1 is held by the holder member 2A in the same manner as in the optical probe 10 in FIG. 1.

The holder member 2F includes an inclined surface 2Fd at a position facing the distal end surface 1Fac of the optical fiber 1F inside the opening hole 2Fa. The reflecting coating 3 as a reflector is arranged on the inclined surface 2Fd. The inclined surface 2Fd and the reflecting surface of the reflecting coating 3 are inclined by a predetermined angle with respect to the optical axis of the optical fiber 1F.

The reflecting coating 3 functions as the traveling direction changing means that changes the traveling direction of the laser beam L output from the optical fiber 1F to a sideward direction with respect to the optical fiber 1F. In the present embodiment, the reflecting coating 3 reflects the laser beam L that travels in an inclined direction with respect to the optical axis of the optical fiber 1F after being output, and changes the traveling direction of the laser beam L such that the traveling direction forms an angle of approximately 90 degrees with the optical axis of the optical fiber 1F. To realize this, the inclination angle of the inclined surface 2Fd is set to be a gradual inclination angle as compared to the inclined surface 2d of the holder member 2 in FIG. 1.

According to an optical probe 10F, it is possible to change the traveling direction of the laser beam L with a simple, small, and easily manufacturable configuration. In particular, the reflecting coating 3 is arranged inside the opening hole 2Fa without protruding to an outer diameter side of the holder member 2F, so that it is possible to reduce an outer diameter of the optical probe 10F.

### Eighth Embodiment

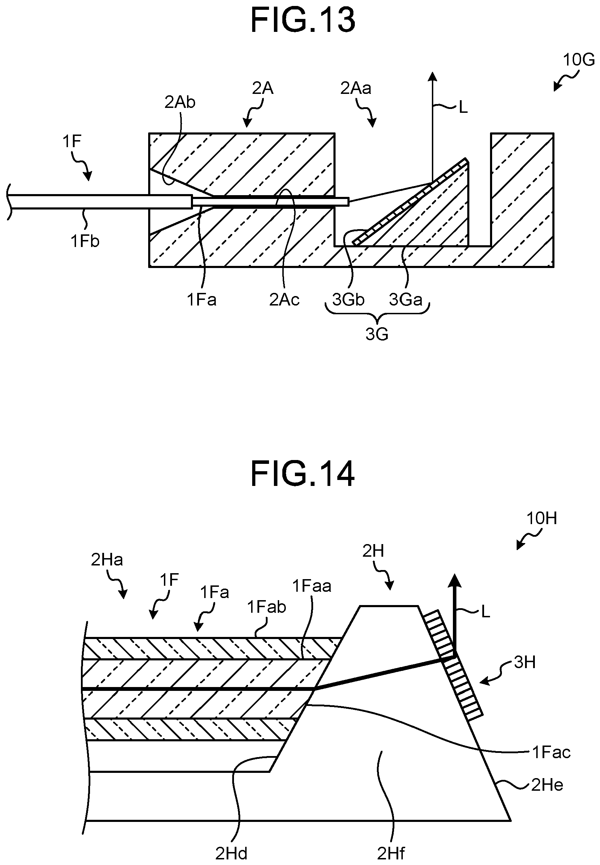

FIG. 13 is a schematic diagram illustrating an overall configuration of an optical probe according to an eighth embodiment. An optical probe 10G has a configuration that is obtained by, in the configuration of the optical probe 10A in FIG. 2, replacing the optical fiber 1 with the optical fiber 1F and replacing the reflecting member 3A with a reflecting member 3G.

The reflecting member 3G is arranged at a position facing the distal end surface of the optical fiber 1F inside the opening hole 2Aa. The reflecting member 3G includes a member 3Ga that is made of glass or the like and that has a certain shape, such as a triangular prism or a tetrahedron, and a reflecting coating 3Gb that is arranged on one surface of the member 3Ga. The reflecting coating 3Gb functions as the traveling direction changing means that changes the traveling direction of the laser beam L output from the optical fiber 1F to a sideward direction with respect to the optical fiber 1F. In the present embodiment, the reflecting coating 3Gb reflects the laser beam L that travels in an inclined direction with respect to the optical axis of the optical fiber 1F after being output, and changes the traveling direction of the laser beam L such that the traveling direction forms an angle of approximately 90 degrees with the optical axis of the optical fiber 1F. To realize this, an inclination angle of the reflecting coating 3Gb is set to be a gradual inclination angle as compared to the reflecting coating 3Ab in FIG. 2.

According to the optical probe 10F, it is possible to change the traveling direction of the laser beam L with a simple, small, and easily manufacturable configuration. In particular, a reflecting coating 3Fb is arranged inside the opening hole 2Aa without protruding to the outer diameter side of the holder member 2A, so that it is possible to reduce an outer diameter of the optical probe 10F.

### Ninth Embodiment

FIG. 14 is a schematic diagram illustrating an overall configuration of an optical probe according to a ninth embodiment. An optical probe 10H includes the optical fiber 1F, a holder member 2H, and a diffraction grating plate 3H.

The holder member 2H is mounted on the distal end side of the optical fiber 1F. The holder member 2H has an approximately cylindrical outer shape and is made of glass in the present embodiment, but a constituent material is not limited to glass as long as it transmits the laser beam L at desired transmissivity. A diameter of the holder member 2 is, for example, approximately 1 to 2 mm or smaller.

The holder member 2H includes an opening hole 2Ha, an optical fiber input hole (not illustrated), and an insertion hole (not illustrated). The optical fiber input hole and the insertion hole respectively have the same configurations as the optical fiber input hole 2b and the insertion hole 2c in FIG. 1, and therefore, explanation thereof will be omitted appropriately. The opening hole 2Ha communicates with the insertion hole, and is opened on a side surface in a direction in which the insertion hole extends, that is, on a cylindrical outer periphery of the holder member 2H.

The holder member 2H includes an inclined surface 2Hd at a position facing the distal end surface 1Fac of the optical fiber 1F in the opening hole 2Ha. The optical fiber 1F is held by the holder member 2A in the same manner as in the optical probe 10 in FIG. 1 such that the distal end surface 1Fac of the optical fiber 1F comes into contact with the inclined surface 2Hd. It is preferable to form an antireflection coating for the laser beam L on the inclined surface 2Hd.

Further, the holder member 2H includes an inclined surface 2He as a distal end surface on the right side in the figure. The inclined surface 2Hd and the inclined surface 2He are inclined in different directions, and a cross section of a distal end portion 2Hf of the holder member 2H has a trapezoidal shape.

The diffraction grating plate 3H is arranged on the inclined surface 2He. In the present embodiment, the diffraction grating plate 3H is a transmissive type. It is preferable to form an antireflection coating for the laser beam L on a surface of a member, such as the inclined surface 2He of the holder member 2H or a surface that comes into contact with the holder member 2H of the diffraction grating plate 3H, through which the laser beam L passes.

The diffraction grating plate 3H functions as the traveling direction changing means that changes the traveling direction of the laser beam L output from the optical fiber 1F to a sideward direction with respect to the optical fiber 1F. Specifically, in the present embodiment, the diffraction grating plate 3H diffracts the laser beam L that travels in an inclined direction with respect to an optical axis of the optical fiber 1F after being output, and changes the traveling direction such that the traveling direction forms an angle of approximately 90 degrees with the optical axis of the optical fiber 1F. In the present embodiment, arrangement orientation of a diffraction grating in the diffraction grating plate 3H is set so as to be parallel to a plane formed by the optical paths of the laser beam L before and after being output from the optical fiber 1F.

According to the optical probe 10H, it is possible to change the traveling direction of the laser beam L with a simple, small, and easily manufacturable configuration. In particular, the diffraction grating plate 3H is arranged so as not to protrude to an outer diameter side of the holder member 2H, so that it is possible to reduce an outer diameter of the optical probe 10H.

### Manufacturing Method

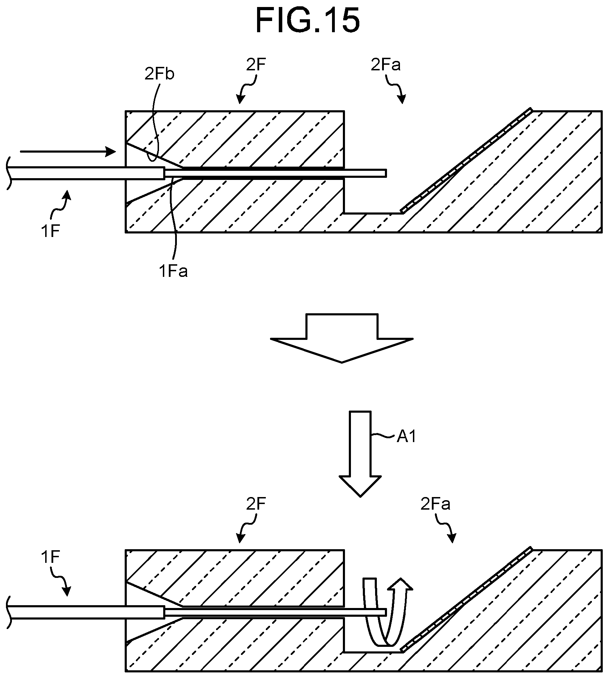

One example of a method of manufacturing the optical probe 10F according to the seventh embodiment illustrated in FIG. 12A and FIG. 12B will be described below with reference to FIG. 15. First, the optical fiber 1F is inserted into the holder member 2F from the optical fiber input hole 2Fb and is inserted in the insertion hole 2Fc, and a relative position of the optical fiber 1F with respect to the holder member 2F is adjusted. Subsequently, after the relative position reaches a predetermined position, rotational alignment is performed by rotating the optical fiber 1F about an axis of the holder member 2F while monitoring a distal end of the optical fiber 1F in the direction of the arrow A1. The distal end surface 1Fac of the optical fiber 1F is inclined, and therefore may serve as a positioning key in the rotational alignment. Further, at the same time or after the rotational alignment, it may be possible to finely adjust the relative position of the optical fiber 1F with respect to the holder member 2F such that the optical path of the laser beam L matches a desired optical path. After completion of the rotational alignment and the fine adjustment, the holder member 2F and the optical fiber 1F are fixed to each other.

The optical probe 10G according to the eighth embodiment illustrated in FIG. 13 can easily be manufactured in the same manner as the simple manufacturing method as illustrated in FIG. 15. Further, as for a method of manufacturing the optical probe 10H according to the ninth embodiment illustrated in FIG. 14, for example, the rotational alignment is first performed on the optical fiber 1F, and the distal end surface 1Fac and the inclined surface 2Hd of the holder member 2H are brought into contact with each other in a parallel manner. At this time, the distal end surface 1Fac and the inclined surface 2Hd may be bonded together. Accordingly, a rotation position of the distal end surface 1Fac is fixed. Thereafter, it is sufficient to determine a position of the diffraction grating plate 3H at a predetermined position on the inclined surface 2He and fix the diffraction grating plate 3H at this position.

### Tenth Embodiment

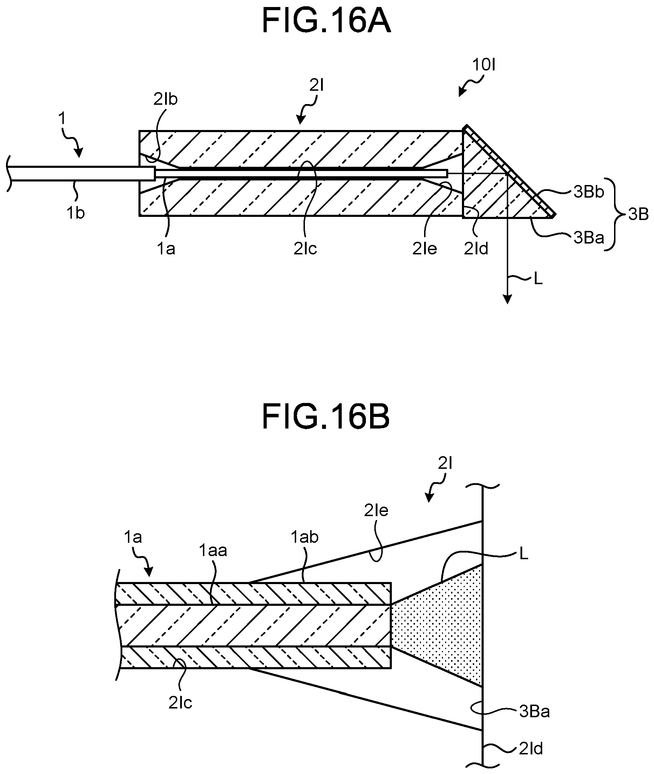

FIG. 16A and FIG. 16B are schematic diagrams illustrating an overall configuration of an optical probe according to a tenth embodiment. As illustrated in FIG. 16A, an optical probe 10I includes the optical fiber 1, a holder member 2I, and the reflecting member 3B.

The holder member 2I includes an optical fiber input hole 2Ib, an insertion hole 2Ic, a diameter extending hole 2Ie, and an end face 2Id. The optical fiber input hole 2Ib and the insertion hole 2Ic respectively have the same configurations as the optical fiber input hole 2Bb and the insertion hole 2Bc of the holder member 2B illustrated in FIG. 3, and therefore, explanation thereof will be omitted appropriately. The diameter extending hole 2Ie is arranged on the end face 2Id of the holder member 2I located on the right side in the figure, and communicates with the insertion hole 2Ic. The diameter extending hole 2Ie has a larger inner diameter than the insertion hole 2Ic. Specifically, the diameter extending hole 2Ie is formed such that the inner diameter is gradually increased from the side communicating with the insertion hole 2Ic toward the end face 2Id. The reflecting member 3B is arranged on the end face 2Id of the holder member 2I similarly to the case illustrated in FIG. 3. A configuration and functions of the reflecting member 3B are the same as those of the third embodiment illustrated in FIG. 3, and therefore, explanation thereof will be omitted appropriately.

Here, as illustrated in FIG. 16A and FIG. 16B, the distal end surface of the optical fiber 1 is located at the side of the optical fiber input hole 2Ib relative to the end face 2Id of the holder member 2I, and is located at a boundary of the insertion hole 2Ic and the diameter extending hole 2Ie or at the side of the diameter extending hole 2Ie relative to the boundary. In the present embodiment, specifically, the distal end surface is located at the side of the diameter extending hole 2Ie relative to the boundary. As illustrated in FIG. 16B, the glass optical fiber 1a includes a core portion 1aa and a cladding portion 1ab, and a beam diameter of the laser beam L is extended after the laser beam L is output from the core portion 1aa. The diameter extending hole 2Ie functions to prevent the laser beam L from being blocked by the holder member 2I even if the beam diameter of the laser beam L is extended as described above. Therefore, an inner diameter of the diameter extending hole 2Ie is set to a certain inner diameter such that the laser beam L is not blocked by the holder member 2I by taking into account NA (the number of openings) of the glass optical fiber 1a, a distance between the distal end surface of the glass optical fiber 1a and the end face 2Id or the like.

### Eleventh Embodiment

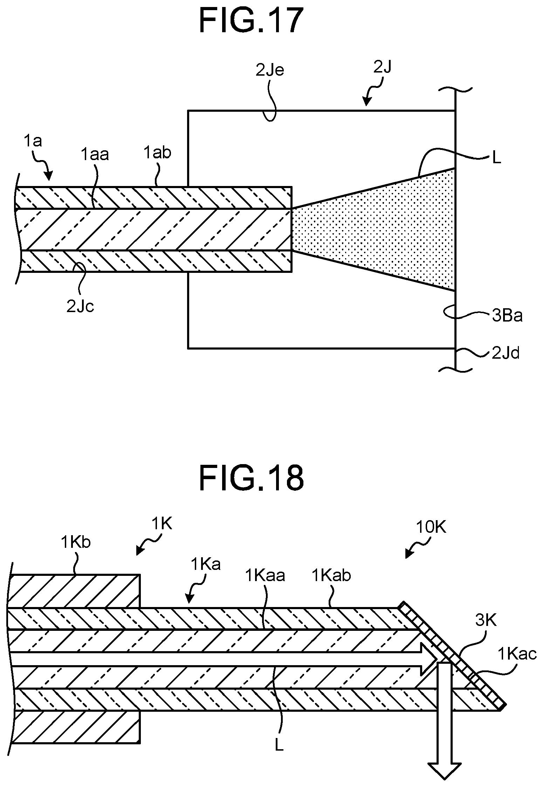

FIG. 17 is a schematic diagram illustrating an overall configuration of an optical probe according to an eleventh embodiment. The optical probe according to the eleventh embodiment is obtained by replacing the holder member 2I with a holder member 2J in the optical probe 10I according to the tenth embodiment illustrated in FIG. 16B. In the holder member 2J, a diameter extending hole 2Je is arranged on an end face 2Jd of the holder member 2J and communicates with an insertion hole 2Jc. The diameter extending hole 2Je has an inner diameter that is larger than that of the insertion hole 2Jc and that is approximately constant in an extending direction of the diameter extending hole 2Je. The diameter extending hole 2Je functions to prevent the laser beam L whose beam diameter is extended after being output from the core portion 1aa from being blocked by the holder member 2J, and the inner diameter is set to implement this function.

### Twelfth Embodiment

FIG. 18 is a schematic diagram illustrating an overall configuration of an optical probe according to a twelfth embodiment. As illustrated in FIG. 18, an optical probe 10K includes an optical fiber 1K and a reflecting coating 3K.

The optical fiber 1K includes a glass optical fiber 1Ka having a core portion 1Kaa and a cladding portion 1Kab, and a covering 1Kb that is formed on an outer circumference of the glass optical fiber 1Ka. In the optical fiber 1K, the covering 1Kb is removed on a distal end side, and a predetermined length of the glass optical fiber 1Ka is exposed. The optical fiber 1K has the same configuration as the optical fiber 1 except that a distal end surface 1Kac from which the laser beam L is output is inclined with respect to an optical axis of the optical fiber 1K, that is, an optical axis of the glass optical fiber 1Ka, and therefore, explanation thereof will be omitted appropriately. The distal end surface 1Kac is inclined by approximately 45 degrees with respect to a plane perpendicular to the optical axis of the optical fiber 1K. The inclination angle as described above can easily be formed by a fiber cutter, mechanical polishing, chemical etching or the like.

The reflecting coating 3K as a reflector is arranged on the distal end surface 1Kac. The reflecting coating 3K is configured with a metal film, a dielectric multi-layer or the like. The reflecting coating 3K functions as the traveling direction changing means that changes the traveling direction of the laser beam L output from the optical fiber 1K to a sideward direction with respect to the optical fiber 1K. In the present embodiment, the reflecting coating 3K reflects the laser beam L, and changes the traveling direction of the laser beam L by approximately 90 degrees.

According to the optical probe 10K, it is possible to change the traveling direction of the laser beam L with a simple, small, and easily manufacturable configuration. In particular, the reflecting coating 3K is arranged on the distal end surface 1Kac of the optical fiber 1K, so that it is possible to reduce an outer diameter of the optical probe 10K and reduce the number of use components.

### Thirteenth Embodiment

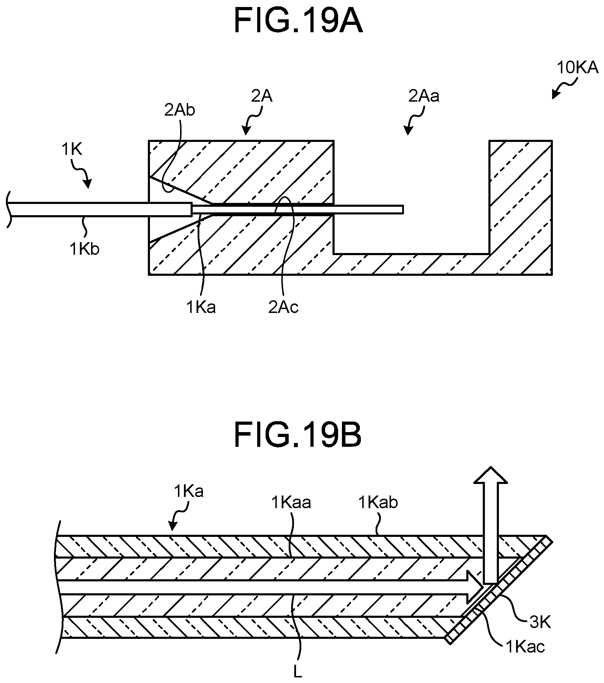

FIG. 19A and FIG. 19B are schematic diagrams illustrating an overall configuration of an optical probe according to a thirteenth embodiment. As illustrated in FIG. 19A and FIG. 19B, an optical probe 10KA is configured by inserting the optical fiber 1K of the optical probe 10K of the twelfth embodiment from the optical fiber input hole 2Ab of the holder member 2A illustrated in FIG. 2, inserting the optical fiber 1K in the insertion hole 2Ac such that the distal end protrudes to the inside of the opening hole 2Aa, and fixing the optical fiber 1K to the holder member 2A. As illustrated in FIG. 19B, in the optical fiber 1K, the distal end surface 1Kac of the optical fiber 1K is oriented to a side opposite to the opening side of the opening hole 2Aa. With this configuration, the reflecting coating 3K reflects the laser beam L, changes the traveling direction of the laser beam L by approximately 90 degrees, and outputs the laser beam L from the opening hole 2Aa.

According to the optical probe 10KA, it is possible to change the traveling direction of the laser beam L with a simple, small, and easily manufacturable configuration. In particular, the reflecting coating 3K is arranged on the distal end surface 1Kac of the optical fiber 1K, so that it is possible to reduce an outer diameter of the optical probe 10KA and reduce the number of use components. Further, it is possible to protect the distal end surface of the optical fiber 1K by the holder member 2A.

### Manufacturing Method

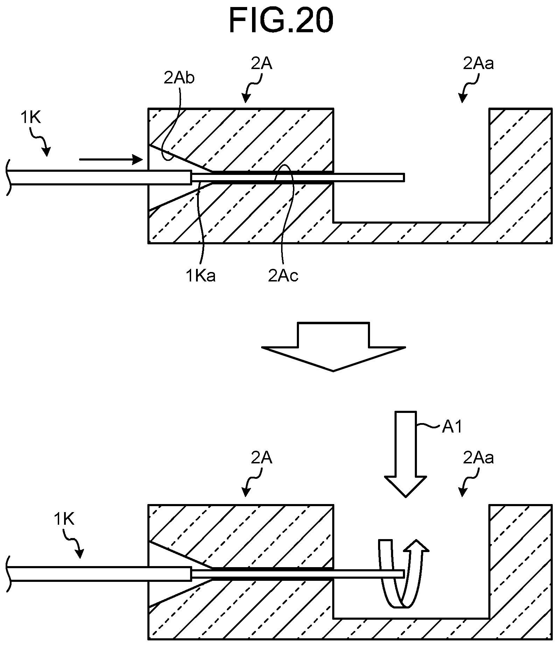

One example of a method of manufacturing the optical probe 10K according to the thirteenth embodiment illustrated in FIG. 19A and FIG. 19B will be described below with reference to FIG. 20. First, the optical fiber 1K is inserted into the holder member 2A from the optical fiber input hole 2Ab and is inserted in the insertion hole 2Ac, and a relative position of the optical fiber 1K with respect to the holder member 2A is adjusted. Subsequently, after the relative position reaches a predetermined position, the rotational alignment is performed by rotating the optical fiber 1K about the axis of the holder member 2A while monitoring a distal end of the optical fiber 1K in the direction of the arrow A1. The distal end surface 1Kac of the optical fiber 1K is inclined, and therefore serves as a positioning key in the rotational alignment. Further, at the same time or after the rotational alignment, it may be possible to finely adjust the relative position of the optical fiber 1K with respect to the holder member 2A such that the optical path of the laser beam L matches a desired optical path. After completion of the rotational alignment and the fine adjustment, the holder member 2A and the optical fiber 1K are fixed to each other.

### Configuration examples of optical fiber

Meanwhile, in the optical probe according to each of the embodiments as described above, in some cases, a monitoring beam with a wavelength different from that of the laser beam L may be input in addition to the laser beam L from a proximal end side of the optical fiber in order to detect flexure or bend of the optical fiber that transmits the laser beam L. In this case, it is desirable to provide, on the distal end side of the optical fiber, a reflecting mechanism that reflects the monitoring beam transmitted in the optical fiber to the proximal end side. Configuration examples of the optical fiber including the reflecting mechanism as described above will be described below.

### First configuration example

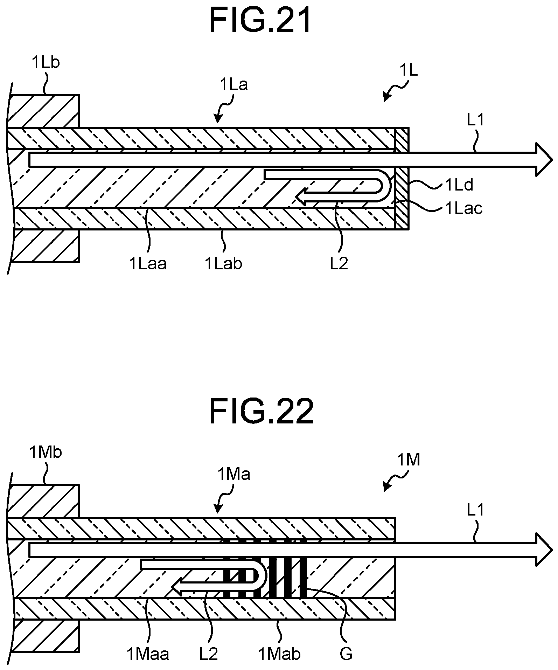

FIG. 21 is a schematic diagram illustrating an overall configuration of a first configuration example of an optical fiber. An optical fiber 1L includes a glass optical fiber 1La having a core portion 1Laa and a cladding portion 1Lab, and a covering 1Lb that is formed on an outer circumference of the glass optical fiber 1La. In the optical fiber 1L, the covering 1Lb is removed on a distal end side, and a predetermined length of the glass optical fiber 1La is exposed. The glass optical fiber 1La has the same configuration as the glass optical fiber 1a illustrated in FIG. 1, and therefore, explanation thereof will be omitted appropriately.

A reflecting coating 1Ld as a reflector is arranged on a distal end surface 1Lac of the glass optical fiber 1La. The reflecting coating 1Ld is, for example, a dielectric multi-layer.

The optical fiber 1L transmits the laser beam L1 in the glass optical fiber 1La. The laser beam L1 is, for example laser beam for cautery. Further, the optical fiber 1L transmits monitoring beam L2 in the glass optical fiber 1La. A wavelength of the monitoring beam L2 is different from a wavelength of the laser beam L1, and is separated by, for example, 3 nm or more. For example, the wavelength of the laser beam L1 belongs to the 980-nm wavelength range, and the monitoring beam L2 belongs to the visible region, the O band, or the C band. The O band is, for example, a wavelength range of 1260 nm to 1360 nm. The C band is, for example, a wavelength range of 1530 nm to 1565 nm.

Here, the reflecting coating 1Ld transmits the laser beam L1. Accordingly, the laser beam L1 is output by being transmitted through the reflecting coating 1Ld. In contrast, the reflecting coating 1Ld reflects the monitoring beam L2 to the proximal end side. Accordingly, the monitoring beam L2 is output from the proximal end side, and is used to detect flexure or bend of the optical fiber 1L. It is preferable to set reflectivity of the reflecting coating 1Ld with respect to the monitoring beam L2 to 4% or higher, and it is more preferable to set the reflectivity to 40% or higher.

The optical fiber 1L is configured in an integrated manner with the reflecting coating 1Ld that serves as a reflecting mechanism, and therefore is configured with a small size. The optical fiber 1L as described above can be used instead of the optical fiber 1 of the embodiment as described above, for example.

### Second configuration example

FIG. 22 is a schematic diagram illustrating an overall configuration of a second configuration example of an optical fiber. An optical fiber 1M includes a glass optical fiber 1Ma having a core portion 1Maa and a cladding portion 1Mab, and a covering 1Mb that is formed on an outer circumference of the glass optical fiber 1Ma. In the optical fiber 1M, the covering 1Mb is removed on a distal end side, and a predetermined length of the glass optical fiber 1Ma is exposed. The glass optical fiber 1Ma has the same configuration as the glass optical fiber 1a illustrated in FIG. 1, and therefore, explanation thereof will be omitted appropriately.

A Bragg grating G as a reflector is arranged in the core portion 1Maa on the distal end side of the glass optical fiber 1Ma. The Bragg grating G is configured such that a refractive index is periodically changed along a longitudinal direction of the core portion 1Maa.

The optical fiber 1M transmits the laser beam L1 and the monitoring beam L2 in the glass optical fiber 1Ma. Here, the Bragg grating G transmits the laser beam L1. Accordingly, the laser beam L1 is output by being transmitted through the Bragg grating G. In contrast, the Bragg grating G reflects the monitoring beam L2 to the proximal end side. Accordingly, the monitoring beam L2 is output from the proximal end side and can be used to detect flexure or bend of the optical fiber 1M. It is preferable to set reflectivity of the Bragg grating G with respect to the monitoring beam L2 to 4% or higher, and it is more preferable to set the reflectivity to 40% or higher.

The optical fiber 1M incorporates therein the Bragg grating G that serves as a reflecting mechanism, and therefore is configured with a small size. The optical fiber 1M as described above can be used instead of the optical fiber 1 of the embodiments as described above, for example.

### Fourteenth Embodiment

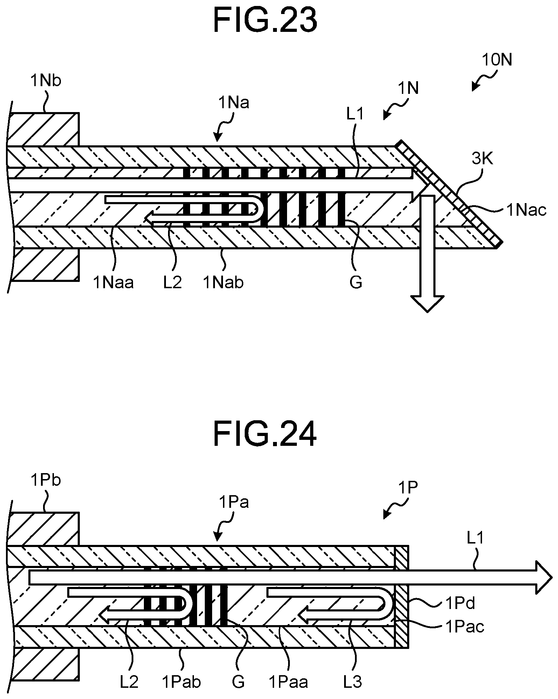

A configuration for reflecting a beam using the Bragg grating can preferably be applied to a configuration in which a distal end surface of an optical fiber is inclined. FIG. 23 is a schematic diagram illustrating an overall configuration of an optical probe according to a fourteenth embodiment. An optical probe 10N includes an optical fiber 1N and the reflecting coating 3K.

The optical fiber 1N includes a glass optical fiber 1Na having a core portion 1Naa and a cladding portion 1Nab, and a covering 1Nb that is formed on an outer circumference of the glass optical fiber 1Na. In the optical fiber 1N, the covering 1Nb is removed on a distal end side, and a predetermined length of the glass optical fiber 1Na is exposed. The optical fiber 1N has the same configuration as the optical fiber 1M except that a distal end surface 1Nac from which the laser beam L1 is output is inclined with respect to an optical axis of the optical fiber 1N, that is, an optical axis of the glass optical fiber 1Na, and therefore, explanation thereof will be omitted appropriately. In other words, the Bragg grating G as a reflector is arranged in the core portion 1Naa on the distal end side of the glass optical fiber 1Na. Meanwhile, the distal end surface 1Nac is inclined by approximately 45 degrees with respect to a plane perpendicular to an optical axis of the optical fiber 1N, and includes the reflecting coating 3K as a reflector.

The optical fiber 1N transmits the laser beam L1 and the monitoring beam L2 in the glass optical fiber 1Na. Here, the Bragg grating G transmits the laser beam L1. Accordingly, the laser beam L1 is output by being transmitted through the Bragg grating G. The reflecting coating 3K reflects the laser beam L1 output from the optical fiber 1N, and changes the traveling direction of the laser beam L by approximately 90 degrees.

In contrast, the Bragg grating G reflects the monitoring beam L2 to the proximal end side. Accordingly, the monitoring beam L2 is output from the proximal end side and can be used to detect flexure and bend of the optical fiber 1N.

### Third configuration example

FIG. 24 is a schematic diagram illustrating an overall configuration of a third configuration example of the optical fiber. An optical fiber 1P includes a glass optical fiber 1Pa having a core portion 1Paa and a cladding portion 1Pab, and a covering 1Pb that is formed on an outer circumference of the glass optical fiber 1Pa. In the optical fiber 1P, the covering 1Pb is removed on a distal end side, and a predetermined length of the glass optical fiber 1Pa is exposed.

A reflecting coating 1Pd as a reflector is arranged on a distal end surface 1Pac of the glass optical fiber 1Pa. The reflecting coating 1Pd is, for example, a dielectric multi-layer. The Bragg grating G as a reflector is arranged in the core portion 1Paa on the distal end side of the glass optical fiber 1Pa.

The optical fiber 1P transmits the laser beam L1, the monitoring beam L2, and monitoring beam L3 in the glass optical fiber 1Pa. A wavelength of the monitoring beam L3 is different from the wavelength of the laser beam L1, and is separated by, for example, 3 nm or more. Further, the wavelength of the monitoring beam L3 is also different from the wavelength of the monitoring beam L2. For example, the wavelength of the laser beam L1 belongs to the 980-nm wavelength range, and the monitoring beam L3 belongs to the visible region, the O band, or the C band.

The Bragg grating G and the reflecting coating 1Pd transmit the laser beam L1. Accordingly, the laser beam L1 is output by being transmitted through the Bragg grating G and the reflecting coating 1Pd. In contrast, the Bragg grating G transmits the monitoring beam L3 and reflects the monitoring beam L2 to the proximal end side. In contrast, the reflecting coating 1Pd reflects the monitoring beam L3 to the proximal end side. Accordingly, the monitoring beam L2 and L3 are output from the proximal end side and can be used to detect flexure or bend of the optical fiber 1P.

The optical fiber 1P is configured in an integrated manner with the Bragg grating G and the reflecting coating 1Pd that serve as reflecting mechanisms, and therefore is configured with a small size. The optical fiber 1P as described above can be used instead of the optical fiber 1 of the embodiments as described above.

Meanwhile, the configuration of the optical fiber including the reflecting mechanism is not limited to the configuration examples as described above, but it may be possible to include, in the core portion, a plurality of Bragg gratings that reflect different wavelengths. Further, it may be possible to form reflecting coatings with characteristics that reflect different wavelengths on a distal end surface of an optical fiber.

Furthermore, in the optical probe according to each of the embodiments as described above, the traveling direction of the laser beam output from the optical fiber is changed by approximately 90 degrees, but the changed traveling direction of a beam is not limited to 90 degrees but may be, for example, in a range of 45 degrees to 135 degrees with respect to the optical axis of the optical fiber.

Moreover, in the optical probe according to each of the embodiments as described above, it may be possible to input what is called an aiming beam from the proximal end side of the optical fiber in the optical probe in order to check a position, such as an affected area, to be irradiated with the laser beam L. As the aiming beam, in general, a visible beam is used. The aiming beam is output from the distal end of the optical fiber similarly to the laser beam L.

The present invention is not limited by the embodiments as described above. The present invention includes configurations that are obtained by appropriately combining constituent elements of each of the embodiments as described above. Furthermore, additional effects and modifications may be easily derived by a person skilled in the art. Therefore, broader aspects of the present invention are not limited to the embodiments as described above, and various modifications may be made.

### Industrial Applicability

An optical probe according to the present invention is useful for an optical probe on a distal end side of an optical fiber that is used in a catheter to be inserted into a body of a patient.

### Reference Signs List

1, 1F, 1K, 1L, 1M, 1N, 1P optical fiber
1a, 1Fa, 1Ka, 1La, 1Ma, 1Na, 1Pa glass optical fiber
1aa, 1Faa, 1Kaa, 1Laa, 1Maa, 1Naa, 1Paa core portion
1ab, 1Fab, 1Kab, 1Lab, 1Mab, 1Nab, 1Pab cladding portion
1b, 1Fb, 1Kb, 1Lb, 1Mb, 1Nb, 1Pb cladding
1Fac, 1Kac, 1Lac, 1Nac, 1Pac distal end surface
1Ld, 1Pd, 3, 3Ab, 3Bb, 3Fb, 3Gb, 3K reflecting coating
2, 2A, 2B, 2D, 2E, 2F, 2H, 2I, 2J holder member
2a, 2Aa, 2Da, 2Fa, 2Ha opening hole
2b, 2Ab, 2Bb, 2Db, 2Eb, 2Fb, 2Ib optical fiber input hole
2c, 2Ac, 2Bc, 2Dc, 2Ec, 2Fc, 2Ic, 2Jc insertion hole
2Bd, 2Id, 2Jd end face
2Dd, 2Ee, 3Ca reflecting surface
2Ed projection portion
2d, 2Fd, 2Hd, 2He inclined surface
2Hf distal end portion
2Ie, 2Je diameter extending hole
3A, 3B, 3C, 3G reflecting member
3Aa, 3Ba, 3Ga member
3H diffraction grating plate
10, 10A, 10B, 10C, 10D, 10E, 10F, 10G, 10H, 10I, 10K, 10KA, 10N optical probe
A1 arrow
G Bragg grating
L, L1 laser beam
L2, L3 monitoring beam
R1, R2, R3 reflecting surface

## Claims

1. An optical probe (10) comprising:
an optical fiber (1),
a holder member (2) that is mounted on a distal end side of the optical fiber (1) and holds the optical fiber (1), wherein the holder member (2) includes an insertion hole (21c) and the optical fiber (1) is inserted in the insertion hole (21c);
a traveling direction changing unit (3) configured to change a traveling direction of an output beam to a sideward direction with respect to the optical fiber (1),
and a reflecting mechanism (1Pd) that is arranged on a distal end surface of the optical fiber (1) from which the output beam is to be output, configured to transmit the output beam and to reflect a monitoring beam with a certain wavelength different from a wavelength of the output beam,
wherein the output beam is for cautery,
wherein the traveling direction changing unit (3) is a reflector that is joined to a part of a surface of the holder member (2) and configured to reflect the output beam, and
wherein the output beam is configured to be output through the reflecting mechanism (lPd), and the reflected monitoring beam is configured to be output from a proximal end side of the optical fiber (1) to facilitate detection of a bend of the optical fiber (1) by the monitoring beam.

2. An optical probe (10) comprising:
an optical fiber (1),
a holder member (2D) that is mounted on a distal end side of the optical fiber (1) and holds the optical fiber (1), wherein the holder member (2D) includes an insertion hole (21c) and the optical fiber (1) is inserted in the insertion hole (21c),
a traveling direction changing unit (2Dd) configured to change a traveling direction of an output beam to a sideward direction with respect to the optical fiber (1),
and a reflecting mechanism (lPd) that is arranged on a distal end surface of the optical fiber (1) from which the output beam is to be output, configured to transmit the output beam and to reflect a monitoring beam with a certain wavelength different from a wavelength of the output beam,
wherein the output beam is for cautery,
wherein the traveling direction changing unit (2Dd) is a part of the holder member (2D) and is configured with a reflecting portion configured to reflect the output beam, and
wherein the output beam is configured to be output through the reflecting mechanism (lPd), and the reflected monitoring beam is configured to be output from a proximal end side of the optical fiber (1) to facilitate detection of a bend of the optical fiber (1) by the monitoring beam.

3. An optical probe (10) comprising:
an optical fiber (1) comprising a glass optical fiber (INa) and a covering (INb),
a holder member (2) that is mounted on a distal end side of the optical fiber (1) and holds the optical fiber (1), wherein the holder member (2) includes an insertion hole (21c) and the optical fiber (1) is inserted in the insertion hole (21c),
a traveling direction changing unit (3K) arranged on an end face of the optical fiber (1) and configured to change a traveling direction of an output beam to a sideward direction with respect to the optical fiber (1);
wherein the output beam is for cautery,
wherein the optical fiber (1) includes a Bragg grating (G) configured to transmit the output beam and to reflect a monitoring beam with a certain wavelength different from a wavelength of the output beam,
wherein the Bragg grating (G) is located in a distal part of the optical fiber (1) in which the covering (1Nb) is removed and the glass optical fiber (1Na) is exposed,
wherein the output beam is configured to be output through the Bragg grating (G), and the reflected monitoring beam is configured to be output from a proximal end side of the optical fiber (1) to facilitate detection of a bend of the optical fiber (1) by the monitoring beam.

4. The optical probe (10) according to claim 1 or 2, wherein
the traveling direction changing unit (3) is a diffraction grating that is arranged on the holder member and diffracts the output beam.

5. The optical probe (10) according to any one of claims 1 to 3, wherein a distal end surface of the optical fiber (1) from which the output beam is output is inclined with respect to an optical axis of the optical fiber (1).

6. The optical probe (10) according to any one of claims 1 to 3, further comprising:
a Bragg grating (G) that is arranged in a core portion of the optical fiber (1), that transmits the beam, and that reflects monitoring beam with a certain wavelength different from a wavelength of the beam.

7. The optical probe (10) according to claim 3, wherein
a distal end surface of the optical fiber (1) from which the output beam is output is inclined with respect to an optical axis of the optical fiber (1), and
the traveling direction changing unit (3K) is a reflector that is arranged on the distal end surface and that reflects the output beam.

8. The optical probe (10) according to claim 7,
wherein
the distal end surface protrudes to an inside of the opening hole (2Aa), and
the distal end surface of the optical fiber (1) is oriented to a side opposite to an opening side of the opening hole (2Aa).

9. The optical probe (10) according to claim 1 or 2, wherein the holder member (2) has an approximately cylindrical outer shape.

10. The optical probe (10) according to any one of claims 1, 2, 3 and 6, wherein the reflecting mechanism or the Bragg grating has reflectivity of 4% or higher with respect to the monitoring beam.

11. The optical probe (10) according to any one of claims 1, 2, 3 and 6, wherein the reflecting mechanism or the Bragg grating has reflectivity of 40% or higher with respect to the monitoring beam.

12. The optical probe (10) according to any one of claims 1, 2, 3, 6, 10 and 11, wherein the certain wavelength of the monitoring beam is separated by 3 nanometers or more from the wavelength of the output beam.

13. The optical probe (10) according to any one of claims 1, 2, 3, 6, 10, 11 and 12, further comprising:
a plurality of reflecting mechanism or Bragg gratings configured to reflect a plurality of monitoring beams.

14. The optical probe (10) according to any one of claims 1, 2, 3, 6, 10, 11, 12 and 13, wherein
the wavelength of the output beam belongs to a 980-nanometer wavelength range, and
the monitoring beam belongs to one of a visible region, an O band, and a C band.

15. The optical probe (10) according to any one of claims 1 to 3, wherein a core diameter of the optical fiber (1) is 65 micrometers or larger.

## Patentansprüche

1. Eine optische Sonde (10) mit:
einer optischen Faser (1),
einem Halterungselement (2), das an einem distalen Ende der optischen Faser (1) angebracht ist und die optische Faser (1) hält, wobei das Halterungselement (2) eine Einführöffnung (21c) aufweist und die optische Faser (1) in die Einführöffnung (21c) eingeführt ist;
eine Laufrichtungsänderungseinheit (3), die dazu eingerichtet ist, eine Laufrichtung eines Ausgangsstrahls in eine seitliche Richtung in Bezug auf die optische Faser (1) zu ändern,
und einen Reflektionsmechanismus (1Pd), der an einer distalen Endfläche der optischen Faser (1) angeordnet ist, von der der Ausgangsstrahl ausgegeben werden soll, und dazu eingerichtet ist, den Ausgangsstrahl zu übertragen und einen Überwachungsstrahl mit einer bestimmten Wellenlänge, die sich von einer Wellenlänge des Ausgangsstrahls unterscheidet, zu reflektieren,
wobei der Ausgangsstrahl zur Kauterisation dient,
wobei die Laufrichtungsänderungseinheit (3) ein Reflektor ist, der mit einem Teil einer Oberfläche des Halterungselements (2) verbunden und dazu eingerichtet ist, den Ausgangsstrahl zu reflektieren, und
wobei der Ausgangsstrahl dazu eingerichtet ist, durch den Reflektionsmechanismus (lPd) ausgegeben zu werden, und der reflektierte Überwachungsstrahl dazu eingerichtet ist, von einer proximalen Endseite der optischen Faser (1) ausgegeben zu werden, um die Erkennung einer Biegung der optischen Faser (1) durch den Überwachungsstrahl zu erleichtern.

2. Eine optische Sonde (10), umfassend:
eine optische Faser (1),
ein Halterungselement (2D), das an einer distalen Endseite der optischen Faser (1) angebracht ist und die optische Faser (1) hält, wobei das Halterungselement (2D) eine Einführöffnung (21c) aufweist und die optische Faser (1) in die Einführöffnung (21c) eingeführt ist,
eine Laufrichtungsänderungseinheit (2Dd), die dazu eingerichtet ist, eine Laufrichtung eines Ausgangsstrahls in eine seitliche Richtung in Bezug auf die optische Faser (1) zu ändern,
und einen Reflektionsmechanismus (lPd), der an einer distalen Endfläche der optischen Faser (1) angeordnet ist, von der der Ausgangsstrahl ausgegeben werden soll, und dazu eingerichtet ist, den Ausgangsstrahl zu übertragen und einen Überwachungsstrahl mit einer bestimmten Wellenlänge, die sich von einer Wellenlänge des Ausgangsstrahls unterscheidet, zu reflektieren,
wobei der Ausgangsstrahl zur Kauterisation dient,
wobei die Laufrichtungsänderungseinheit (2Dd) ein Teil des Halterungselements (2D) ist und mit einem Reflektionsabschnitt versehen ist, der dazu eingerichtet ist, den Ausgangsstrahl zu reflektieren, und
wobei der Ausgangsstrahl dazu eingerichtet ist, durch den Reflektionsmechanismus (lPd) ausgegeben zu werden, und der reflektierte Überwachungsstrahl dazu eingerichtet ist, von einer proximalen Endseite der optischen Faser (1) ausgegeben zu werden, um die Erkennung einer Biegung der optischen Faser (1) durch den Überwachungsstrahl zu erleichtern.

3. Eine optische Sonde (10), umfassend:
eine optische Faser (1), die eine Glasoptikfaser (INa) und eine Hülle (INb) umfasst,
ein Halterungselement (2), das an einer distalen Endseite der optischen Faser (1) angebracht ist und die optische Faser (1) hält, wobei das Halterungselement (2) eine Einführöffnung (21c) umfasst und die optische Faser (1) in die Einführöffnung (21c) eingeführt ist,
eine Laufrichtungsänderungseinheit (3K), die an einer Endfläche der optischen Faser (1) angeordnet und dazu eingerichtet ist, eine Laufrichtung eines Ausgangsstrahls in eine seitliche Richtung in Bezug auf die optische Faser (1) zu ändern;
wobei der Ausgangsstrahl zur Kauterisation dient,
wobei die optische Faser (1) ein Bragg-Gitter (G) umfasst, das dazu eingerichtet ist, den Ausgangsstrahl zu übertragen und einen Überwachungsstrahl mit einer bestimmten Wellenlänge, die sich von einer Wellenlänge des Ausgangsstrahls unterscheidet, zu reflektieren,
wobei das Bragg-Gitter (G) in einem distalen Teil der optischen Faser (1) angeordnet ist, in dem die Hülle (1Nb) entfernt ist und die Glasoptikfaser (1Na) freiliegt,
wobei der Ausgangsstrahl dazu eingerichtet ist, durch das Bragg-Gitter (G) ausgegeben zu werden, und der reflektierte Überwachungsstrahl dazu eingerichtet ist, von einer proximalen Endseite der optischen Faser (1) ausgegeben zu werden, um die Erkennung einer Biegung der optischen Faser (1) durch den Überwachungsstrahl zu erleichtern.

4. Die optische Sonde (10) nach Anspruch 1 oder 2, wobei
die Laufrichtungsänderungseinheit (3) ein Beugungsgitter ist, das auf dem Halterungselement angeordnet ist und den Ausgangsstrahl beugt.

5. Die optische Sonde (10) nach einem der Ansprüche 1 bis 3, wobei eine distale Endfläche der optischen Faser (1), von der der Ausgangsstrahl ausgegeben wird, in Bezug auf eine optische Achse der optischen Faser (1) geneigt ist.

6. Die optische Sonde (10) nach einem der Ansprüche 1 bis 3, ferner umfassend:
ein Bragg-Gitter (G), das in einem Kernbereich der optischen Faser (1) angeordnet ist, den Strahl durchlässt und einen Überwachungsstrahl mit einer bestimmten Wellenlänge, die sich von einer Wellenlänge des Strahls unterscheidet, reflektiert.

7. Die optische Sonde (10) nach Anspruch 3, wobei
eine distale Endfläche der optischen Faser (1), von der der Ausgangsstrahl ausgegeben wird, in Bezug auf eine optische Achse der optischen Faser (1) geneigt ist, und
die Laufrichtungsänderungseinheit (3K) ein Reflektor ist, der auf der distalen Endfläche angeordnet ist und den Ausgangsstrahl reflektiert.

8. Die optische Sonde (10) nach Anspruch 7, wobei
die distale Endfläche in das Innere des Öffnungslochs (2Aa) hineinragt und
die distale Endfläche der optischen Faser (1) zu einer Seite hin ausgerichtet ist, die einer Öffnungsseite des Öffnungslochs (2Aa) gegenüberliegt.

9. Die optische Sonde (10) nach Anspruch 1 oder 2, wobei das Halterungselement (2) eine annähernd zylindrische Außenform aufweist.

10. Die optische Sonde (10) nach einem der Ansprüche 1, 2, 3 und 6, wobei der Reflektionsmechanismus oder das Bragg-Gitter eine Reflektivität von 4 % oder mehr in Bezug auf den Überwachungsstrahl aufweist.

11. Die optische Sonde (10) nach einem der Ansprüche 1, 2, 3 und 6, wobei der Reflektionsmechanismus oder das Bragg-Gitter eine Reflektivität von 40 % oder mehr in Bezug auf den Überwachungsstrahl aufweist.

12. Die optische Sonde (10) nach einem der Ansprüche 1, 2, 3, 6, 10 und 11, wobei die Wellenlänge des Überwachungsstrahls um 3 Nanometer oder mehr von der Wellenlänge des Ausgangsstrahls abweicht.

13. Die optische Sonde (10) nach einem der Ansprüche 1, 2, 3, 6, 10, 11 und 12, die ferner umfasst:
mehrere Reflektionsmechanismen oder Bragg-Gitter, die dazu eingrichtet sind, die mehreren Überwachungsstrahlen zu reflektieren.

14. Die optische Sonde (10) nach einem der Ansprüche 1, 2, 3, 6, 10, 11, 12 und 13, wobei
die Wellenlänge des Ausgangsstrahls in einen Wellenlängenbereich von 980 Nanometern fällt und
die Wellenlänge des Überwachungsstrahls zu einem aus einem sichtbaren Bereich, einem O-Band und einem C-Band gehört.

15. Die optische Sonde (10) nach einem der Ansprüche 1 bis 3, wobei der Kerndurchmesser der optischen Faser (1) 65 Mikrometer oder größer ist.

## Revendications

1. Sonde optique (10) comprenant :
une fibre optique (1),
un élément de maintien (2) qui est monté sur un côté extrémité distale de la fibre optique (1) et maintient la fibre optique (1), l'élément de maintien (2) incluant un orifice d'insertion (21c) et la fibre optique (1) étant insérée dans l'orifice d'insertion (21c) ;
une unité de modification de direction de déplacement (3) configurée pour modifier une direction de déplacement d'un faisceau de sortie vers une direction latérale par rapport à la fibre optique (1),
et un mécanisme réfléchissant (1Pd) qui est agencé sur une surface d'extrémité distale de la fibre optique (1) à partir de laquelle le faisceau de sortie doit être fourni en sortie, configuré pour transmettre le faisceau de sortie et pour réfléchir un faisceau de surveillance avec une certaine longueur d'onde différente d'une longueur d'onde du faisceau de sortie,
dans laquelle le faisceau de sortie est destiné à la cautérisation,
dans laquelle l'unité de modification de la direction de déplacement (3) est un réflecteur relié à une partie d'une surface de l'élément de maintien (2) et configuré pour réfléchir le faisceau de sortie, et
dans laquelle le faisceau de sortie est configuré pour être fourni en sortie à travers le mécanisme réfléchissant (1Pd), et le faisceau de surveillance réfléchi est configuré pour être fourni en sortie à partir d'un côté extrémité proximale de la fibre optique (1) afin de faciliter la détection d'une courbure de la fibre optique (1) par le faisceau de surveillance.

2. Sonde optique (10) comprenant :
une fibre optique (1),
un élément de maintien (2D) qui est monté sur un côté extrémité distale de la fibre optique (1) et maintient la fibre optique (1), l'élément de maintien (2D) incluant un orifice d'insertion (21c) et la fibre optique (1) étant insérée dans l'orifice d'insertion (21c),
une unité de modification de direction de déplacement (2Dd) configurée pour modifier une direction de déplacement d'un faisceau de sortie vers une direction latérale par rapport à la fibre optique (1),
et un mécanisme réfléchissant (1Pd) qui est agencé sur une surface d'extrémité distale de la fibre optique (1) à partir de laquelle le faisceau de sortie doit être fourni en sortie, configuré pour transmettre le faisceau de sortie et pour réfléchir un faisceau de surveillance avec une certaine longueur d'onde différente d'une longueur d'onde du faisceau de sortie,
dans laquelle le faisceau de sortie est destiné à la cautérisation,
dans laquelle l'unité de modification de la direction de déplacement (2Dd) fait partie de l'élément de maintien (2D) et est configurée avec une portion réfléchissante configurée pour réfléchir le faisceau de sortie, et
dans laquelle le faisceau de sortie est configuré pour être fourni en sortie à travers le mécanisme réfléchissant (1Pd), et le faisceau de surveillance réfléchi est configuré pour être fourni en sortie à partir d'un côté extrémité proximale de la fibre optique (1) afin de faciliter la détection d'une courbure de la fibre optique (1) par le faisceau de surveillance.

3. Sonde optique (10) comprenant :
une fibre optique (1) comprenant une fibre optique en verre (1Na) et un revêtement (1 Nb), un élément de maintien (2) qui est monté sur un côté extrémité distale de la fibre optique (1) et maintient la fibre optique (1), l'élément de maintien (2) incluant un orifice d'insertion (21c) et la fibre optique (1) étant insérée dans l'orifice d'insertion (21c),
une unité de modification de direction de déplacement (3K) agencée sur une face d'extrémité de la fibre optique (1) et configurée pour modifier une direction de déplacement d'un faisceau de sortie vers une direction latérale par rapport à la fibre optique (1) ;
dans laquelle le faisceau de sortie est destiné à la cautérisation,
dans laquelle la fibre optique (1) inclut un réseau de Bragg (G) configuré pour transmettre le faisceau de sortie et pour réfléchir un faisceau de surveillance avec une certaine longueur d'onde différente d'une longueur d'onde du faisceau de sortie,
dans laquelle le réseau de Bragg (G) est situé dans une partie distale de la fibre optique (1) dans laquelle le revêtement (1Nb) est enlevé et la fibre optique en verre (1Na) est exposée,
dans laquelle le faisceau de sortie est configuré pour être fourni en sortie à travers le réseau de Bragg (G), et le faisceau de surveillance réfléchi est configuré pour être fourni en sortie à partir d'un côté extrémité proximale de la fibre optique (1) afin de faciliter la détection d'une courbure de la fibre optique (1) par le faisceau de surveillance.

4. Sonde optique (10) selon la revendication 1 ou 2, dans laquelle
l'unité de modification de direction de déplacement (3) est un réseau de diffraction qui est agencé sur l'élément de maintien et qui diffracte le faisceau de sortie.

5. Sonde optique (10) selon l'une quelconque des revendications 1 à 3, dans laquelle une surface d'extrémité distale de la fibre optique (1) à partir de laquelle le faisceau de sortie est fourni en sortie est inclinée par rapport à un axe optique de la fibre optique (1).

6. Sonde optique (10) selon l'une quelconque des revendications 1 à 3, comprenant en outre :
un réseau de Bragg (G) qui est agencé dans une portion de cœur de la fibre optique (1), qui transmet le faisceau et qui réfléchit le faisceau de surveillance avec une certaine longueur d'onde différente d'une longueur d'onde du faisceau.

7. Sonde optique (10) selon la revendication 3, dans laquelle
une surface d'extrémité distale de la fibre optique (1) à partir de laquelle le faisceau de sortie est fourni en sortie est inclinée par rapport à un axe optique de la fibre optique (1), et
l'unité de modification de direction de déplacement (3K) est un réflecteur qui est agencé sur la surface d'extrémité distale et qui réfléchit le faisceau de sortie.

8. Sonde optique (10) selon la revendication 7,
dans laquelle
la surface d'extrémité distale fait saillie à l'intérieur de l'orifice d'ouverture (2Aa), et
la surface d'extrémité distale de la fibre optique (1) est orientée vers un côté opposé à un côté ouverture de l'orifice d'ouverture (2Aa).

9. Sonde optique (10) selon la revendication 1 ou 2, dans laquelle l'élément de maintien (2) présente une forme extérieure approximativement cylindrique.

10. Sonde optique (10) selon l'une quelconque des revendications 1, 2, 3 et 6, dans laquelle le mécanisme réfléchissant ou le réseau de Bragg présente une réflectivité égale ou supérieure à 4 % par rapport au faisceau de surveillance.

11. Sonde optique (10) selon l'une quelconque des revendications 1, 2, 3 et 6, dans laquelle le mécanisme réfléchissant ou le réseau de Bragg présente une réflectivité égale ou supérieure à 40 % par rapport au faisceau de surveillance.

12. Sonde optique (10) selon l'une quelconque des revendications 1, 2, 3, 6, 10 et 11, dans laquelle la longueur d'onde du faisceau de surveillance est séparée de 3 nanomètres ou plus de la longueur d'onde du faisceau de sortie.

13. Sonde optique (10) selon l'une quelconque des revendications 1, 2, 3, 6, 10, 11 et 12, comprenant en outre :
une pluralité de mécanismes réfléchissants ou de réseaux de Bragg configurés pour réfléchir une pluralité de faisceaux de surveillance.

14. Sonde optique (10) selon l'une quelconque des revendications 1, 2, 3, 6, 10, 11, 12 et 13, dans laquelle la longueur d'onde du faisceau de sortie appartient à une plage de longueurs d'onde de 980 nanomètres, et la longueur d'onde du faisceau de surveillance appartient à l'une parmi une région visible, une bande O et une bande C.

15. Sonde optique (10) selon l'une quelconque des revendications 1 à 3, dans laquelle le diamètre de cœur de la fibre optique (1) est supérieur ou égal à 65 micromètres.
